# EUROPEAN PATENT APPLICATION

(11) **EP 4 279 494 A1**
(43) Date of publication of application: **22.11.2023**
(21) Application number: 21906417.7
(22) Date of filing: 06.12.2021
(51) Int. Cl.: C07F 7/02, A61K 31/409, A61K 31/695, A61K 41/00, A61P 9/00, A61P 17/00, A61P 17/06, A61P 17/10, A61P 31/04, A61P 35/00

(54) **METHOD FOR DECOMPOSING COMPOUND, AND COMPOUND**

(30) Priority: 16.12.2020 JP 2020208367
(71) Applicant: National University Corporation Hokkaido University, Sapporo-shi, Hokkaido 060-0808 (JP)
(72) Inventor: OGAWA, Mikako, Sapporo-shi, Hokkaido 060-0808 (JP); TAKAKURA, Hideo, Sapporo-shi, Hokkaido 060-0808 (JP); MATSUHIRO, Shino, Sapporo-shi, Hokkaido 060-0808 (JP); INANAMI, Osamu, Sapporo-shi, Hokkaido 060-0808 (JP)
(74) Representative: Grünecker Patent- und Rechtsanwälte PartG mbB
(86) International application number: PCT/JP2021/044732
(87) International publication number: WO 2022/131054

(57) **Abstract**

An object is to provide a method for decomposing a photosensitive dye with an energy beam capable of penetrating deeper into a living body than near-infrared light and activating the photosensitive dye and a photosensitive dye having high photosensitivity. Provided is a method for decomposing a compound represented by Formula (1), the method including irradiating the compound with X-rays in a presence of an electron donor: 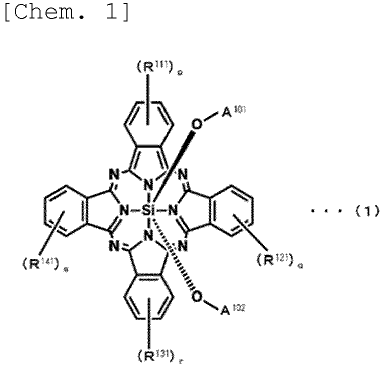 (in Formula (1), R¹¹¹, R¹²¹, R¹³¹, and R¹⁴¹ each independently represent a monovalent organic group; p, q, r, and s each independently represent an integer of 0 or 1 to 4; in the case where two or more R¹¹¹, R¹²¹, R¹³¹, and R¹⁴¹ are present, they may be identical to or different from one another or may be bonded to one another to form a ring; A¹⁰¹ represents a monovalent organic group; and A¹⁰² represents a hydrogen atom or a monovalent organic group).

## Description

### Technical Field

The present invention relates to a method for decomposing a compound and a compound.

### Background Art

A novel therapy that uses an antibody and a photosensitive dye, that is, photo immunotherapy, which is particularly used as a cancer therapy, is described in PTLs 1 and 2. Attention has been focused on photoimmunotherapy as a therapy that kills or damages target cells (cancer cells) by irradiation with near-infrared light and has few side effects.

The photosensitive dye used in photoimmunotherapy is IRDye 700DX represented by Formula (I) below (product name, produced by LI-COR, Inc.; may be referred to as "IR700"). It is considered that the adequate wavelength of the light that excites IR700 is 660 to 740 nm.

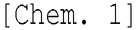

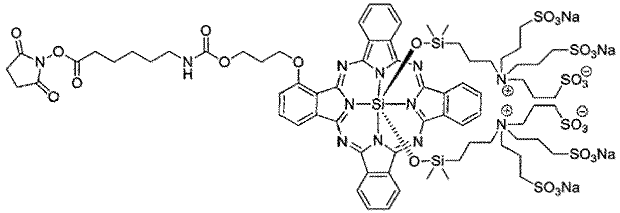

Upon being exposed to near-infrared light (e.g., 690 ± 20 nm), IR700 becomes decomposed to convert from hydrophilic to hydrophobic and consequently becomes aggregated. This enables the cell membranes of target cells to be damaged and induces cytotoxicity due to apoptosis, necrosis, and/or autophagy in the target cells.

Although the wavelengths of near-infrared light fall within a wavelength range in which tissue permeability is relatively high, near-infrared light can penetrate only the surface portion of a human body because the attenuation of the light occurs due to scattering or absorption of the light. Although a method of delivering the light deep into a living body with a catheter or the like has been studied, a method of noninvasively activating a chemical present in deep parts of a living body is more preferable.

It is considered that establishing a method for decomposing a photosensitive dye with an energy beam capable of penetrating deeper into a living body than near-infrared light and activating the photosensitive dye enables the noninvasive activation of a chemical present in deep parts of a living body. It is also considered that the noninvasive activation of a chemical present in deep parts of a living body can also be achieved by developing a highly photosensitive dye (chemical) capable of killing or damaging cells similarly to IR700 even when irradiated with a light beam (energy beam) having high tissue permeability other than near-infrared light or weaker light.

However, there has not been sufficient knowledge about the molecular design of a photosensitive dye other than IR700 which enables the development of a chemical capable of producing its advantageous effects even when irradiated with weak light.

The inventors of the present invention found that, in photoimmunotherapy, the dissociation of the axial ligands of IR700 is important for producing the effects of killing or damaging cells and made a report in NPL 1.

In NPL 2, the inventors of the present invention also announced that the results of the quantum chemical calculation conducted by the inventors of the present invention confirmed that the dissociation of axial ligands is considered likely to occur in a compound including a phthalocyanine ring the axial ligands of which are likely to be protonated in an excited state.

### Citation List

### Patent Literature

PTL 1: Japanese Unexamined Patent Application Publication (Translation of PCT Application) No. 2017-524002
PTL 2: Japanese Unexamined Patent Application Publication (Translation of PCT Application) No. 2017-524659

### Non Patent Literature

NPL 1: ACS Cent. Sci. 2018, 4, 1559-69.
NPL 2: Chem Plus Chem. 2020, 85, 1959-1963.

### Summary of Invention

### Technical Problem

An object that is to be achieved in the present invention is to provide a method for decomposing a photosensitive dye with an energy beam capable of penetrating deeper into a living body than near-infrared light and activating the photosensitive dye and a photosensitive dye having high photosensitivity.

### Solution to Problem

The inventors of the present invention conducted extensive studies in order to achieve the above object and consequently found that the above object can be achieved by irradiating a specific compound (photosensitive dye) with X-rays in the presence of an electron donor and by using a compound having a specific structure. Thus, the present invention was made.

The inventors of the present invention synthesized compounds that included silicon phthalocyanine skeletons and various axial ligands other than IR700 which were introduced to the silicon phthalocyanine skeletons and found that there was a correlation between the progress of the axial ligand dissociation reaction and the likelihood of protonation of the axial ligands. It was also found that, in the presence of an electron donor, such as water, the axial ligands of the above compounds can be easily dissociated by not only irradiation with near-infrared light but also irradiation with X-rays.

The inventors conducted studies on the basis of the above knowledge, consequently discovered a photosensitive dye the axial ligands of which can be dissociated in the presence of an electron donor, even in the case where the photosensitive dye is irradiated with X-rays instead of near-infrared light, and devised a method for decomposing a compound with X-rays. Since X-rays are capable of penetrating deep into a living body, X-rays may be used as external stimuli that enables drug efficacy to be produced in deep parts of a living body.

It is anticipated that, on the basis of the above knowledge, a photosensitive dye (chemical) having higher photosensitivity than IR700 can be developed.

Specifically, the present invention provides the method for decomposing a compound and the compound which are described below.

Article 1: A method for decomposing a compound represented by Formula (1), the method including irradiating the compound with X-rays in a presence of an electron donor: 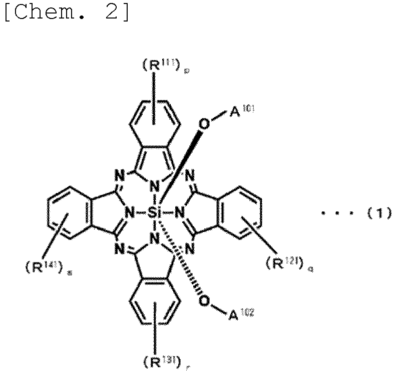 (in Formula (1), R¹¹¹, R¹²¹, R¹³¹, and R¹⁴¹ each independently represent a monovalent organic group; p, q, r, and s each independently represent an integer of 0 or 1 to 4; in the case where two or more R¹¹¹, R¹²¹, R¹³¹, and R¹⁴¹ are present, they may be identical to or different from one another or may be bonded to one another to form a ring; A¹⁰¹ represents a monovalent organic group; and A¹⁰² represents a hydrogen atom or a monovalent organic group).

Article 2: The method for decomposing the compound described in Article 1, wherein a product of the decomposition is one or more compounds selected from the group consisting of a compound represented by Formula (2), a compound represented by Formula (3), and a compound represented by Formula (4).
Formula (2): 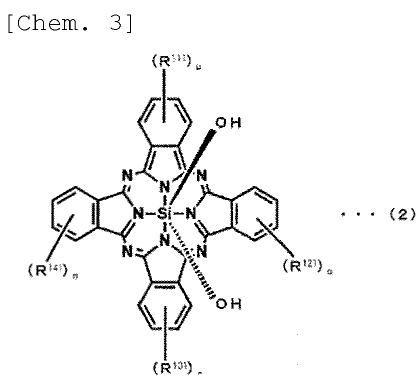 (in Formula (2), R¹, R², R³, R⁴, p, q, r, and s represent the same things as R¹, R², R³, R⁴, p, q, r, and s in Formula (1), respectively)
Formula (3):

A¹⁰¹-OH ··· (3)

(in Formula (3), A¹⁰¹ represents the same thing as A¹⁰¹ in Formula (1))
Formula (4):

A¹⁰²-OH ... (4)

(in Formula (4), A¹⁰² represents the same thing as A¹⁰² in Formula (1))

Article 3: The method for decomposing the compound described in Article 1, wherein the X-rays have wavelengths of 1 pm to 2 nm and/or the X-rays have an irradiation energy of 1 keV to 100 MeV.

Article 4: The method for decomposing the compound described in Article 1 or 2,
wherein the A¹⁰¹ is a group represented by Formula (1a): 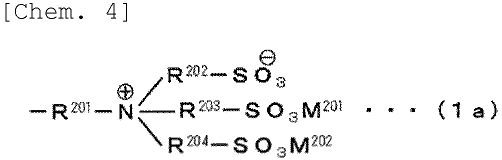 (in Formula (1a), R²⁰¹, R²⁰², R²⁰³, and R²⁰⁴ each independently represent a divalent organic group and may be identical to or different from one another; and M²⁰¹ and M²⁰² each independently represent a monovalent cation and may be identical to or different from one another), and
wherein the A¹⁰² is a hydrogen atom or a group represented by Formula (1b): 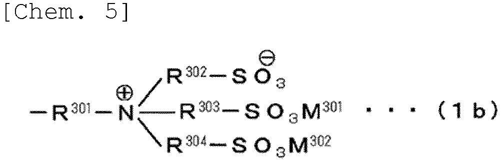 (in Formula (1b), R³⁰¹, R³⁰², R³⁰³, and R³⁰⁴ each independently represent a divalent organic group and may be identical to or different from one another; and M³⁰¹ and M³⁰² each independently represent a monovalent cation and may be identical to or different from one another).

Article 5: The method for decomposing the compound described in any one of Articles 1 to 3, wherein at least one of the R¹¹¹, R¹²¹, R¹³¹, and R¹⁴¹ is a group represented by Formula (1c):

-L-Q ··· (1c)

(in Formula (1c), L represents a divalent organic group, and Q represents a reactive group responsible for addition to a probe).

Article 6: The method for decomposing the compound described in Article 1,
wherein the A¹⁰¹ is any one of groups represented by Formulae (1a₁), (1a₂), and (1a₃),
   Formula (1a₁): 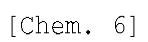 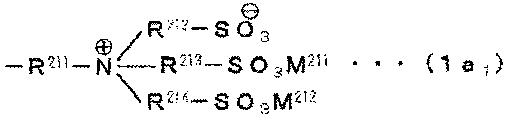 (in Formula (1a₁), R²¹¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R²¹², R²¹³, and R²¹⁴ each independently represent a divalent aliphatic hydrocarbon group having 1 to 20 carbon atoms and may be identical to or different from one another; and M²¹¹ and M²¹² each independently represent an alkali metal ion and may be identical to or different from one another)
   Formula (1a₂): 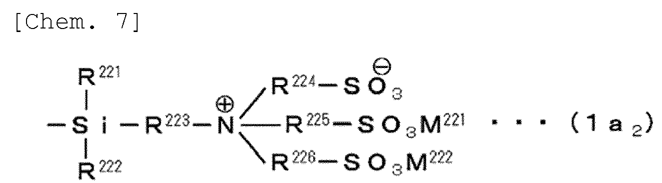 (in Formula (1a₂), R²²¹ and R²²² each independently represent a monovalent aliphatic hydrocarbon group having 1 to 20 carbon atoms or a monovalent aromatic hydrocarbon group having 6 to 30 carbon atoms; R²²³ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R²²⁴, R²²⁵, and R²²⁶ each independently represent a divalent aliphatic hydrocarbon group having 1 to 20 carbon atoms and may be identical to or different from one another; and M²²¹ and M²²² each independently represent an alkali metal ion and may be identical to or different from one another)
   Formula (1a₃): 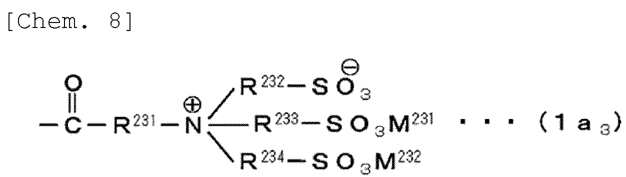 (in Formula (1a₃), R²³¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R²³², R²³³, and R²³⁴ each independently represent a divalent aliphatic hydrocarbon group having 1 to 20 carbon atoms and may be identical to or different from one another; and M²³¹ and M²³² each independently represent an alkali metal ion and may be identical to or different from one another), and
wherein the A¹⁰² is any one of a hydrogen atom and groups represented by Formulae (1b₁), (1b₂), and (1b₃),
   Formula (1b₁): 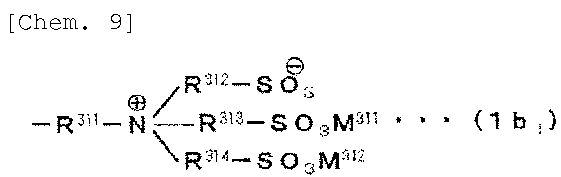 (in Formula (1b₁), R³¹¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R³¹², R³¹³, and R³¹⁴ each independently represent a divalent aliphatic hydrocarbon group having 1 to 20 carbon atoms and may be identical to or different from one another; and M³¹¹ and M³¹² each independently represent an alkali metal ion and may be identical to or different from one another)
   Formula (1b₂): 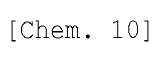 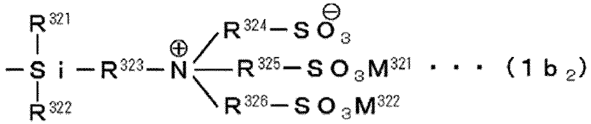 (in Formula (1b₂), R³²¹ and R³²² each independently represent a monovalent aliphatic hydrocarbon group having 1 to 20 carbon atoms or a monovalent aromatic hydrocarbon group having 6 to 30 carbon atoms; R³²³ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R³²⁴, R³²⁵, and R³²⁶ each independently represent a divalent aliphatic hydrocarbon group having 1 to 20 carbon atoms and may be identical to or different from one another; and M³²¹ and M³²² each independently represent an alkali metal ion and may be identical to or different from one another)
   Formula (1b₃): 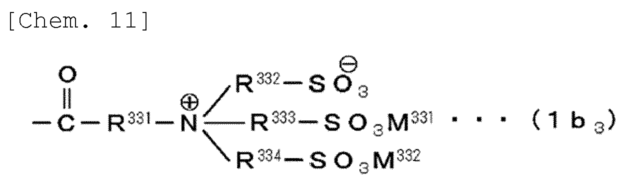 (in Formula (1b₃), R³³¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R³³², R³³³, and R³³⁴ each independently represent a divalent aliphatic hydrocarbon group having 1 to 20 carbon atoms and may be identical to or different from one another; and M³³¹ and M³³² each independently represent an alkali metal ion and may be identical to or different from one another).

Article 7: The method for decomposing the compound described in any one of Articles 1 to 6, the method being conducted in a presence of hydrated electrons and/or carbon dioxide anion radicals.

Article 8: The method for decomposing the compound described in any one of Articles 1 to 7, wherein an amount of X-rays absorbed is less than 10 Gy.

Article 9: A compound represented by Formula (3): 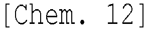 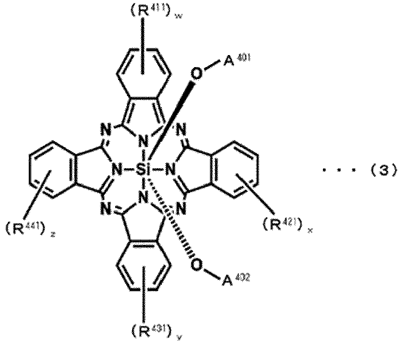 (in Formula (3), R⁴¹¹, R⁴²¹, R⁴³¹, and R⁴⁴¹ each independently represent a monovalent organic group; w, x, y, and z each independently represent an integer of 0 or 1 to 4; in the case where two or more R⁴¹¹, R⁴²¹, R⁴³¹, and R⁴⁴¹ are present, they may be identical to or different from one another or may be bonded to one another to form a ring;
A⁴⁰¹ represents any one of groups represented by Formulae (3a₁), (3a₂), and (3a₃),
   Formula (3a₁): 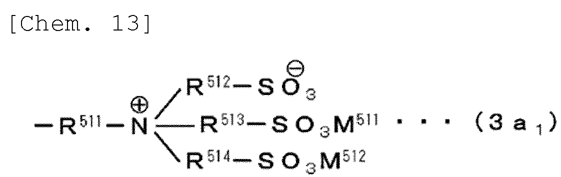 (in Formula (3a₁), R⁵¹¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁵¹², R⁵¹³, and R⁵¹⁴ each independently represent a divalent aliphatic hydrocarbon group having 1 to 20 carbon atoms and may be identical to or different from one another; and M⁵¹¹ and M⁵¹² each independently represent an alkali metal ion and may be identical to or different from one another)
   Formula (3a₂): 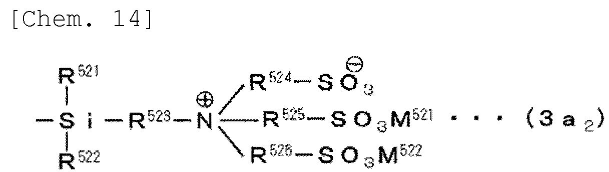 (in Formula (3a₂), R⁵²¹ and R⁵²² each independently represent a monovalent aliphatic hydrocarbon group having 1 to 20 carbon atoms or a monovalent aromatic hydrocarbon group having 6 to 30 carbon atoms; R⁵²³ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁵²⁴, R⁵²⁵, and R⁵²⁶ each independently represent a divalent aliphatic hydrocarbon group having 1 to 20 carbon atoms and may be identical to or different from one another; and M⁵²¹ and M⁵²² each independently represent an alkali metal ion and may be identical to or different from one another)
   Formula (3a₃): 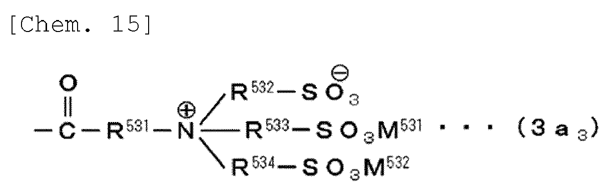 (in Formula (3a₃), R⁵³¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁵³², R⁵³³, and R⁵³⁴ each independently represent a divalent aliphatic hydrocarbon group having 1 to 20 carbon atoms and may be identical to or different from one another; and M⁵³¹ and M⁵³² each independently represent an alkali metal ion and may be identical to or different from one another), and
A⁴⁰² represents any one of a hydrogen atom and groups represented by Formulae (3b₁), (3b₂), and (3b₃),
   Formula (3b₁): 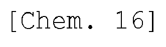 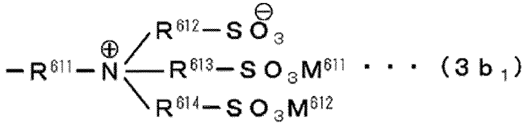 (in Formula (3b₁), R⁶¹¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁶¹², R⁶¹³, and R⁶¹⁴ each independently represent a divalent aliphatic hydrocarbon group having 1 to 20 carbon atoms and may be identical to or different from one another; and M⁶¹¹ and M⁶¹² each independently represent an alkali metal ion and may be identical to or different from one another)
   Formula (3b₂): 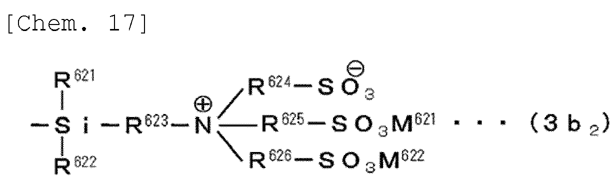 (in Formula (3b₂), R⁶²¹ and R⁶²² each independently represent a monovalent aliphatic hydrocarbon group having 1 to 20 carbon atoms or a monovalent aromatic hydrocarbon group having 6 to 30 carbon atoms; R⁶²³ represents -CH₂-, - CH₂-CH₂-, -CH(CH₃)CH₂-, -CH₂CH(CH₃)-, a divalent aliphatic hydrocarbon group having 4 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁶²⁴, R⁶²⁵, and R⁶²⁶ each independently represent a divalent aliphatic hydrocarbon group having 1 to 20 carbon atoms and may be identical to or different from one another; and M⁶²¹ and M⁶²² each independently represent an alkali metal ion and may be identical to or different from one another)
   Formula (3b₃): 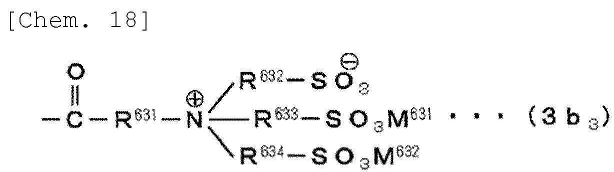 (in Formula (3b₃), R⁶³¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁶³², R⁶³³, and R⁶³⁴ each independently represent a divalent aliphatic hydrocarbon group having 1 to 20 carbon atoms and may be identical to or different from one another; and M⁶³¹ and M⁶³² each independently represent an alkali metal ion and may be identical to or different from one another)).

### Advantageous Effects of Invention

The method for decomposing a compound according to the present invention provides a method for decomposing a photosensitive dye with an energy beam capable of penetrating deeper into a living body than near-infrared light and activating the photosensitive dye. The compound according to the present invention provides a photosensitive dye having high photosensitivity.

### Description of Embodiments

Details of embodiments of the present invention are described below.

It should be understood that the present invention is not limited by the following embodiments and includes various modifications implemented without departing from the scope of the present invention.

### [Method for Decomposing Compound]

A method for decomposing a compound according to a first embodiment of the present invention is a method for decomposing a compound represented by Formula (1), the method including irradiating the compound with X-rays in the presence of an electron donor: 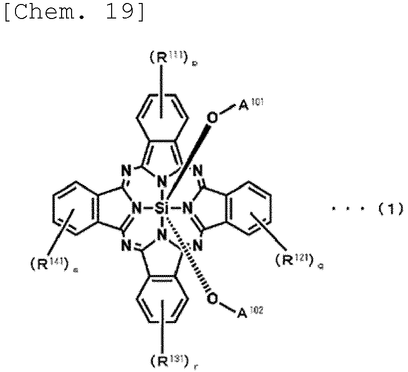 (in Formula (1), R¹¹¹, R¹²¹, R¹³¹, and R¹⁴¹ each independently represent a monovalent organic group; p, q, r, and s each independently represent an integer of 0 or 1 to 4; in the case where two or more R¹¹¹, R¹²¹, R¹³¹, and R¹⁴¹ are present, they may be identical to or different from one another or may be bonded to one another to form a ring; A¹⁰¹ represents a monovalent organic group; and A¹⁰² represents a hydrogen atom or a monovalent organic group). Hereinafter, the compound represented by Formula (1) may be referred to as "compound (1)".

### <Electron Donor>

Examples of the electron donor used in the method for decomposing a compound according to the present invention include molecules and ions that are likely to donate electrons to another. Commonly, a base defined by the Lewis theory of acids and bases can be used as an electron donor.

Examples thereof include oxygen compounds, such as water, alcohols, ethers, and ketones; and nitrogen compounds, such as ammonia and amines.

In the present invention, water and alcohols are preferably used as an electron donor.

### <Compound Represented by Formula (1)>

The compound represented by Formula (1) which is used in the method for decomposing a compound according to the present invention is represented by the following formula: 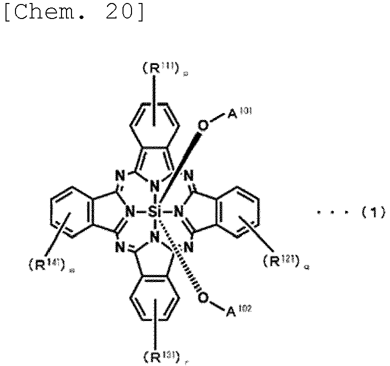 (in Formula (1), R¹¹¹, R¹²¹, R¹³¹, and R¹⁴¹ each independently represent a monovalent organic group; p, q, r, and s each independently represent an integer of 0 or 1 to 4; in the case where two or more R¹¹¹, R¹²¹, R¹³¹, and R¹⁴¹ are present, they may be identical to or different from one another or may be bonded to one another to form a ring; A¹⁰¹ represents a monovalent organic group; and A¹⁰² represents a hydrogen atom or a monovalent organic group).

Preferable examples of the compound represented by Formula (1) include the compound represented by Formula (3) below.

### <X-Rays>

Examples of the X-rays used in the method for decomposing a compound according to the first embodiment of the present invention include an electromagnetic wave having wavelengths of 1 pm to 2 nm. Examples of the X-rays used in the method for decomposing a compound according to the first embodiment of the present invention also include an electromagnetic wave having an irradiation energy of 1 keV to 100 MeV.

Since X-rays have high irradiation energies and high particulate nature, they have a high ability to penetrate living bodies.

As X-rays, any of ultrasoft X-rays (0.01 keV or less), soft X-rays (about 0.1 to 2 keV), X-rays (about 2 to 20 keV), and hard X-rays (20 keV or more) can be used. Among these, hard X-rays are preferably used since they have a high penetration ability and a large irradiation energy.

### <Mechanisms By Which Compound Is Decomposed>

The inventors of the present invention consider that, in the case where water is used as an example of the electron donor in the method for decomposing a compound according to the first embodiment of the invention, the compound (1) is decomposed by the following mechanisms. Note that the following consideration does not limit the present invention. 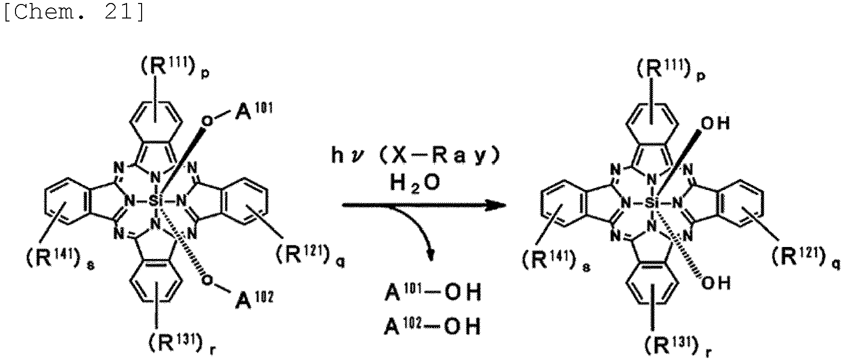 (in the above formula, R¹¹¹, R¹²¹, R¹³¹, R¹⁴¹, p, q, r, s, A¹⁰¹, and A¹⁰² represent the same things as those defined in Formula (1), respectively)

### <Product of Decomposition>

In the method for decomposing a compound according to the first embodiment of the present invention, the compound (1) can be decomposed by being irradiated with X-rays in the presence of an electron donor. As a result of the decomposition of the compound (1), one or more compounds selected from the group consisting of compounds represented by Formulae (2a), (2b), and (2c) can be produced.
Formula (2a): 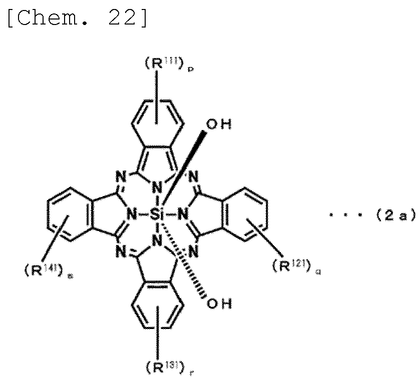 (in Formula (2a), R¹¹¹, R¹²¹, R¹³¹, R¹⁴¹, p, q, r, and s represent the same things as R¹¹¹, R¹²¹, R¹³¹, R¹⁴¹, p, q, r, and s defined in Formula (1), respectively)
Formula (2b):

A¹⁰¹-OH ··· (2b)

(in Formula (2b), A¹⁰¹ represents the same thing as A¹⁰¹ defined in Formula (1))
Formula (2c):

A¹⁰²-OH ... (2c)

(in Formula (2c), A¹⁰² represents the same thing as A¹⁰² defined in Formula (1))

The compound represented by Formula (1) is a compound having higher hydrophilicity than the compound represented by Formula (2a). When the compound represented by Formula (1) is decomposed to the compound represented by Formula (2a) upon the dissociation of the axial ligands, a sudden change from hydrophilicity to hydrophobicity occurs. This causes aggregation of the compound represented by Formula (2a) .

For example, cells can be killed by bonding the compound represented by Formula (1) to the cell membrane, irradiating the cells with X-rays to cause dissociation of axial ligands, and thereby forming aggregates on the cell membrane due to the change in physical property, which induce cell membrane defect.

The compounds represented by Formulae (2b) and/or (2c) correspond to residues of the decomposition of the axial ligands of the compound represented by Formula (1).

For example, in the case where the axial ligands include a chemical bonded thereto, the chemical can be released when the compound represented by Formula (1) is irradiated with X-rays. This enables the use of the compound as a caged compound.

### <Decomposition Conditions>

Examples of the conditions under which the method for decomposing a compound according to the present invention is implemented include the following conditions.

The temperature is, for example, 0°C to 100°C and is preferably room temperature (e.g., 23°C) to 45°C.

The atmosphere is preferably an atmosphere free of oxygen; if oxygen is present, the oxygen may inhibit the decomposition reaction of the compound (1). The decomposition method can be implemented in, for example, an inert gas atmosphere, such as an argon or nitrogen atmosphere. The decomposition method can also be implemented in a nitrous oxide (N₂O) atmosphere.

In the case where water is used as an electron donor, the radiolysis of water occurs upon irradiation with X-rays to produce ·OH (OH radical) and e⁻_{aq} (hydrated electron). The results of studies conducted by the inventors of the present invention confirmed that e⁻_{aq} (hydrated electrons) and/or CO₂⁻· (carbon dioxide anion radicals) accelerate the decomposition reaction (dissociation of the axial ligands) of the compound (1) and that the decomposition reaction is a radical chain reaction. Moreover, on the basis of the results of studies conducted by the inventors of the present invention, it is considered that the ·OH (OH radical) does not greatly contribute to the decomposition reaction (dissociation of the axial ligands) of the compound (1).

For example, in the case where water is used as an electron donor, a water-soluble compound may be dissolved in the water in an amount with which the decomposition reaction is not inhibited.

For example, various water-soluble salts may be dissolved in the water. Examples of the water-soluble salts include an alkali metal salt of an inorganic acid and an alkali metal salt of an organic acid. Examples of the inorganic acid include hydrochloric acid, nitric acid, phosphoric acid, sulfuric acid, and boric acid. Examples of the organic acid include formic acid, acetic acid, tartaric acid, and citric acid. Specific examples thereof include one or more selected from the group consisting of sodium chloride, potassium chloride, sodium nitrate, potassium nitrate, sodium sulfate, potassium sulfate" sodium dihydrogen phosphate, disodium hydrogen phosphate, sodium formate, potassium formate, sodium acetate, potassium acetate, sodium tartrate, and sodium citrate.

In the case where water is used as an electron donor, the pH of the water is preferably, but not limited to, less than 7.0 and preferably falls within an acidic region of 6.5 or less in consideration of the decomposition reaction (dissociation of the axial ligands) of the compound (1).

In the present invention, the amount of the X-rays absorbed can be set to less than 10 Gy, that is, for example, 7 Gy or less, and is preferably set to 5 Gy or less. In the present invention, the decomposition reaction (dissociation of the axial ligands) of the compound (1) can be achieved even when the amount of the X-rays absorbed is less than 10 Gy. Thus, the present invention is markedly useful in consideration of application to human bodies.

### [Compound]

A compound according to a second embodiment of the present invention is specifically a compound represented by Formula (3) below (hereinafter, may be referred to as "compound (3)").

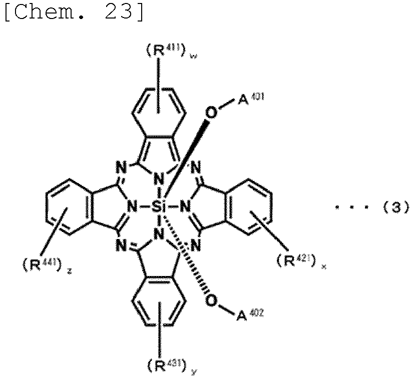

In Formula (3), R⁴¹¹, R⁴²¹, R⁴³¹, and R⁴⁴¹ each independently represent a monovalent organic group; w, x, y, and z each independently represent an integer of 0 or 1 to 4; in the case where two or more R⁴¹¹, R⁴²¹, R⁴³¹, and R⁴⁴¹ are present, they may be identical to or different from one another or may be bonded to one another to form a ring.

The monovalent organic groups represented by R⁴¹¹, R⁴²¹, R⁴³¹, and R⁴⁴¹ are, for example, each independently one or more selected from the group consisting of a hydrocarbon group having 1 to 30 carbon atoms, an alkoxy group, an aryloxy group, a heterocyclic group, a halogen group, a hydroxyl group, a carboxyl group, a carboxylic acid ester group, an amino group, a substituted amino group, a nitro group, a phosphate group, a phosphoric acid ester group, and the like.

The above groups may include one or more substituents. Examples of such substituents include one or more selected from the group consisting of a halogen atom, a nitro group, a carboxyl group, a hydroxyl group, a silyl group, an alkyl group, an alkoxy group having 1 to 3 carbon atoms, a heterocyclic group, an amino group, a thiol group, an acyl group, a phosphate group, and the like.

Examples of the hydrocarbon group include an aliphatic hydrocarbon group, an alicyclic hydrocarbon group, an aromatic hydrocarbon group, an aromatic-aliphatic hydrocarbon group, and an alicyclic-aliphatic hydrocarbon group. These groups may include one or more substituents.

The aliphatic hydrocarbon group is, for example, a linear or branched aliphatic hydrocarbon group having 1 to 30 carbon atoms and is preferably a linear or branched aliphatic hydrocarbon group having 1 to 10 carbon atoms. Specific examples thereof include an alkyl group, an alkenyl group, and an alkynyl group.

The alicyclic hydrocarbon group is, for example, a saturated or unsaturated alicyclic hydrocarbon group having 3 to 30 carbon atoms and is preferably a saturated or unsaturated alicyclic hydrocarbon group having 3 to 12 carbon atoms. Specific examples thereof include a cycloalkyl group, a cycloalkenyl group, and a cycloalkadienyl group.

The aromatic hydrocarbon group is, for example, an aromatic hydrocarbon group having 6 to 30 carbon atoms and is preferably an aryl group having 6 to 14 carbon atoms.

The aromatic-aliphatic hydrocarbon group is, for example, an aromatic-aliphatic hydrocarbon group having 7 to 30 carbon atoms and is preferably an aromatic-aliphatic hydrocarbon group having 7 to 14 carbon atoms. Specific examples thereof include an aralkyl group and an arylalkenyl group.

The alicyclic-aliphatic hydrocarbon group is, for example, a saturated or unsaturated alicyclic-aliphatic hydrocarbon group having 4 to 30 carbon atoms and is preferably an alicyclic-aliphatic hydrocarbon group having 4 to 13 carbon atoms. Specific examples thereof include a cycloalkylalkyl group and a cycloalkylalkenyl group.

The substituent that may be included in the monovalent hydrocarbon group having 1 to 30 carbon atoms may be one or more substituents present at the positions at which the hydrocarbon group may have substituents. Examples of the above substituent include a halogen atom, a nitro group, a carboxyl group, a hydroxyl group, a silyl group, an alkoxy group having 1 to 3 carbon atoms, a heterocyclic group, an amino group, a thiol group, an acyl group, and a phosphate group.

In Formula (3), any one of R⁴¹¹, R⁴²¹, R⁴³¹, and R⁴⁴¹ may be a linker represented by Q-L-, where L represents a divalent organic group and Q represents a reactive group responsible for addition to a probe.

In the case where the compound (3) includes the linker represented by Q-L-, the compound (3) can be bonded to a probe, such as a ligand, a peptide, or a protein, which can be specifically bonded to specific cells. In such a case, the compound (3) can be applied to medical use.

Q of the linker includes a reactive group that reacts with a carboxyl group, an amine, a thiol group, or the like present on a probe and causes the linker to add to the probe. Examples of the reactive group include one or more groups selected from an activated ester group, a halogenated acyl group, a halogenated alkyl group, an amino group that may be substituted, an anhydride group, a carboxyl group, a carbodiimide group, a hydroxyl group, an iodoacetamide group, an isocyanate group, an isothiocyanate group, a maleimide group, an NHS ester group, a phosphoramidite group, a sulfonic acid ester group, a thiol group, a thiocyanate group, and the like.

Examples of the linker represented by Q-L- may include a phosphoramidite group, an NHS ester group, a carboxylic acid, thiocyanate, isothiocyanate, maleimide, and iodoacetamide.

L of the linker may be absent (direct bond). L of the linker may be, for example, a divalent hydrocarbon group having 1 to 30 carbon atoms which may include one or more selected from -O- (ether group), -S- (thioether group), -NH-(amine group), -COO- (ester group), -NHCOO- (urethane group), -NHCONH- (urea group), -NHCSNH- (thiourea group), -NHCO-(amide group), a nitrogen-oxygen bond, a nitrogen-nitrogen bond, an oxygen-phosphorus bond, a sulfur-phosphorus bond, and the like.

Examples of the divalent hydrocarbon group having 1 to 30 carbon atoms include an aliphatic hydrocarbon group, an alicyclic hydrocarbon group, an aromatic hydrocarbon group, an aromatic-aliphatic hydrocarbon group, and an alicyclic-aliphatic hydrocarbon group. These divalent hydrocarbon groups may include one or more substituents.

The aliphatic hydrocarbon group is, for example, a linear or branched aliphatic hydrocarbon group having 1 to 30 carbon atoms and is preferably a linear or branched aliphatic hydrocarbon group having 1 to 10 carbon atoms. Specific examples thereof include an alkylene group, an alkenylene group, and an alkynylene group.

Preferable examples of the alkylene group include alkylene groups having 1 to 10 carbon atoms, such as methylene, ethylene, propylene, isopropylene, butylene, isobutylene, sec-butylene, tert-butylene, pentylene, isopentylene, neopentylene, 1-ethylpropylene, hexylene, 1,1-dimethylbutylene, 2,2-dimethylbutylene, 3,3-dimethylbutylene, 2-ethylbutylene, heptylene, and octylene.

Preferable examples of the alkenylene group include alkenylene groups having 2 to 10 carbon atoms, such as ethenylene, 1-propenylene, 2-propenylene, 2-methyl-1-propenylene, 1-butenylene, 2-butenylene, 3-butenylene, 3-methyl-2-butenylene, 1-pentenylene, 4-methyl-3-pentenylene, 1-hexenylene, 1-heptenylene, and 1-octenylene.

Preferable examples of the alkynylene group include alkynylene groups having 2 to 10 carbon atoms, such as ethynylene, 1-propynylene, 2-propynylene, 1-butynylene, 1-pentynylene, 2-pentynyl, 1-hexynylene, 2-hexynylene, 1-heptynylene, and 1-octynylene.

The alicyclic hydrocarbon group is, for example, a saturated or unsaturated alicyclic hydrocarbon group having 3 to 30 carbon atoms and is preferably a saturated or unsaturated alicyclic hydrocarbon group having 3 to 12 carbon atoms. Specific examples thereof include a cycloalkylene group, a cycloalkenylene group, and a cycloalkadienylene group.

Preferable examples of the cycloalkylene group include cycloalkylene groups having 3 to 12 carbon atoms, such as cyclopropylene, cyclobutylene, cyclopentylene, cyclohexylene, cycloheptylene, cyclooctylene, bicyclo[2.2.1]heptylene, bicyclo[2.2.2]octylene, bicyclo[3.2.1]octylene, bicyclo[3.2.2]nonylene, and bicyclo[4.3.1]decylene.

Preferable examples of the cycloalkenylene group include cycloalkenylene groups having 3 to 12 carbon atoms, such as 2-cyclopentelen-1-yl, 3-cyclopentelen-1-yl, 2-cyclohexelen-1-yl, and 3-cyclohexelen-1-yl.

Preferable examples of the cycloalkadienylene group include cycloalkadienylene groups having 4 to 12 carbon atoms, such as 2,4-cyclopentadielen-1-yl, 2,4-cyclohexadielen-1-yl, and 2,5-cyclohexadielen-1-yl.

The aromatic hydrocarbon group is, for example, an aromatic hydrocarbon group having 3 to 30 carbon atoms and is preferably an arylene group having 6 to 14 carbon atoms. Examples thereof include phenylene, naphthylene, anthrylene, phenanthrylene, acenaphthylenylene, biphenylylene, and indenylene. Among these, phenylene, naphthylene, and the like are preferable. The arylene group may be partially saturated. Examples of the partially saturated arylene group include dihydroindenylene.

The aromatic-aliphatic hydrocarbon group is, for example, an aromatic-aliphatic hydrocarbon group having 3 to 30 carbon atoms and is preferably an aromatic-aliphatic hydrocarbon group having 7 to 14 carbon atoms. Specific examples thereof include an aralkylene group and an arylalkenylene group.

Preferable examples of the aralkylene group include aralkylene groups having 7 to 14 carbon atoms, such as benzylene, phenethylene, phenylpropylene, naphthylmethylene, and benzhydrylene.

Preferable examples of the arylalkenylene group include arylalkenylene groups having 8 to 14 carbon atoms, such as styrylene.

The alicyclic-aliphatic hydrocarbon group is, for example, a saturated or unsaturated alicyclic-aliphatic hydrocarbon group having 3 to 30 carbon atoms and is preferably a saturated or unsaturated alicyclic-aliphatic hydrocarbon group having 4 to 13 carbon atoms. Specific examples thereof include a cycloalkylalkylene group and a cycloalkylalkenylene group.

Preferable examples of the cycloalkylalkylene group include cycloalkylalkylene groups having 4 to 13 carbon atoms, such as cyclopropylmethylene, cyclopropylethylene, cyclopentylmethylene, cyclopentylethylene, cyclohexylmethylene, and cyclohexylethylene.

Preferable examples of the cycloalkylalkenylene group include cycloalkylalkenylene groups having 5 to 13 carbon atoms, such as cyclopropylethenylene, cyclopentylethenylene, and cyclohexylethenylene.

The substituent that may be included in the divalent hydrocarbon group having 1 to 30 carbon atoms may be one or more substituents present at the positions at which the hydrocarbon group may have substituents. Examples of the above substituent include one or more selected from the group consisting of a halogen atom, a nitro group, a carboxyl group, a hydroxyl group, a silyl group, an alkoxy group having 1 to 3 carbon atoms, a heterocyclic group, an amino group, a thiol group, an acyl group, a phosphate group, and the like.

In the present invention, examples of the linker represented by Q-L- include

-O-(CH₂)ₙ-NH₂,

-O-(CH₂)ₙ-COOH,

-O-(CH₂)ₙ-SH,

and
a group represented by the following formula (where * denotes the position at which the group is bonded to the phthalocyanine skeleton).

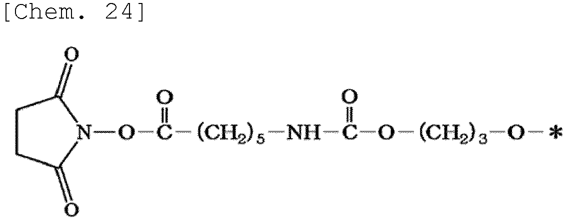

The above linker may include a structure of a sugar, a peptide, a polyalkylene glycol, a nucleotide, or the like.

In Formula (3), A⁴⁰¹ is any of the groups represented by Formulae (3a₁), (3a₂), and (3a₃).

Formula (3a₁) is the group represented by the following formula.

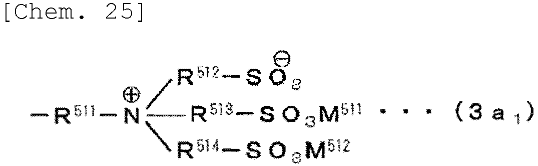

In Formula (3a₁), R⁵¹¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms. These groups may include one or more selected from -O-(ether group), -S- (thioether group), -NH- (amine group), - COO- (ester group), -NHCOO- (urethane group), -NHCONH- (urea group), -NHCSNH- (thiourea group), -NHCO- (amide group), a nitrogen-oxygen bond, a nitrogen-nitrogen bond, an oxygen-phosphorus bond, a sulfur-phosphorus bond, and the like and may have one or more substituents.

The aliphatic hydrocarbon group is, for example, a linear or branched divalent aliphatic hydrocarbon group having 1 to 20 carbon atoms and is preferably a linear or branched aliphatic hydrocarbon group having 1 to 10 carbon atoms. Specific examples thereof include an alkylene group, an alkenylene group, and an alkynylene group.

Preferable examples of the alkylene group include alkylene groups having 1 to 10 carbon atoms, such as methylene, ethylene, propylene, isopropylene, butylene, isobutylene, sec-butylene, tert-butylene, pentylene, isopentylene, neopentylene, 1-ethylpropylene, hexylene, 1,1-dimethylbutylene, 2,2-dimethylbutylene, 3,3-dimethylbutylene, 2-ethylbutylene, heptylene, and octylene.

Preferable examples of the alkenylene group include alkenylene groups having 2 to 10 carbon atoms, such as ethenylene, 1-propenylene, 2-propenylene, 2-methyl-1-propenylene, 1-butenylene, 2-butenylene, 3-butenylene, 3-methyl-2-butenylene, 1-pentenylene, 4-methyl-3-pentenylene, 1-hexenylene, 1-heptenylene, and 1-octenylene.

Preferable examples of the alkynylene group include alkynylene groups having 2 to 10 carbon atoms, such as ethynylene, 1-propynylene, 2-propynylene, 1-butynylene, 1-pentynylene, 2-pentynyl, 1-hexynylene, 2-hexynylene, 1-heptynylene, and 1-octynylene.

The alicyclic hydrocarbon group is, for example, a saturated or unsaturated alicyclic hydrocarbon group having 3 to 20 carbon atoms and is preferably a saturated or unsaturated alicyclic hydrocarbon group having 3 to 12 carbon atoms. Specific examples thereof include a cycloalkylene group, a cycloalkenylene group, and a cycloalkadienylene group.

Preferable examples of the cycloalkylene group include cycloalkylene groups having 3 to 12 carbon atoms, such as cyclopropylene, cyclobutylene, cyclopentylene, cyclohexylene, cycloheptylene, cyclooctylene, bicyclo[2.2.1]heptylene, bicyclo[2.2.2]octylene, bicyclo[3.2.1]octylene, bicyclo[3.2.2]nonylene, and bicyclo[4.3.1]decylene.

Preferable examples of the cycloalkenylene group include cycloalkenylene groups having 3 to 12 carbon atoms, such as 2-cyclopentelen-1-yl, 3-cyclopentelen-1-yl, 2-cyclohexelen-1-yl, and 3-cyclohexelen-1-yl.

Preferable examples of the cycloalkadienylene group include cycloalkadienylene groups having 4 to 12 carbon atoms, such as 2,4-cyclopentadielen-1-yl, 2,4-cyclohexadielen-1-yl, and 2,5-cyclohexadielen-1-yl.

The aromatic hydrocarbon group is, for example, an aromatic hydrocarbon group having 6 to 30 carbon atoms and is preferably an arylene group having 6 to 14 carbon atoms. Examples thereof include phenylene, naphthylene, anthrylene, phenanthrylene, acenaphthylenylene, biphenylylene, and indenylene. Among these, phenylene, naphthylene, and the like are preferable. The arylene group may be partially saturated. Examples of the partially saturated arylene group include dihydroindenylene.

The aromatic-aliphatic hydrocarbon group is, for example, an aromatic-aliphatic hydrocarbon group having 7 to 30 carbon atoms and is preferably an aromatic-aliphatic hydrocarbon group having 7 to 14 carbon atoms. Specific examples thereof include an aralkylene group and an arylalkenylene group.

Preferable examples of the aralkylene group include aralkylene groups having 7 to 14 carbon atoms, such as benzylene, phenethylene, phenylpropylene, naphthylmethylene, and benzhydrylene.

Preferable examples of the arylalkenylene group include arylalkenylene groups having 8 to 14 carbon atoms, such as styrylene.

The alicyclic-aliphatic hydrocarbon group is, for example, a saturated or unsaturated alicyclic-aliphatic hydrocarbon group having 4 to 20 carbon atoms and is preferably a saturated or unsaturated alicyclic-aliphatic hydrocarbon group having 4 to 13 carbon atoms. Specific examples thereof include a cycloalkylalkylene group and a cycloalkylalkenylene group.

Preferable examples of the cycloalkylalkylene group include cycloalkylalkylene groups having 4 to 13 carbon atoms, such as cyclopropylmethylene, cyclopropylethylene, cyclopentylmethylene, cyclopentylethylene, cyclohexylmethylene, and cyclohexylethylene.

Preferable examples of the cycloalkylalkenylene group include cycloalkylalkenylene groups having 5 to 13 carbon atoms, such as cyclopropylethenylene, cyclopentylethenylene, and cyclohexylethenylene.

The substituent may be one or more substituents present at the positions at which the hydrocarbon group may have substituents. Examples of the above substituent include one or more selected from the group consisting of a halogen atom, a nitro group, a carboxyl group, a hydroxyl group, a silyl group, an alkoxy group having 1 to 3 carbon atoms, a heterocyclic group, an amino group, a thiol group, an acyl group, a phosphate group, and the like.

Among these groups, in the case where R⁵¹¹ is an alkylene group having 2 to 10 carbon atoms, the dissociation reaction of the axial ligands can occur in an effective manner even when the amount of light (near-infrared light) or X-rays irradiated/absorbed is small.

In Formula (3a₁), R⁵¹², R⁵¹³, and R⁵¹⁴ each independently represent a divalent aliphatic hydrocarbon group having 1 to 20 carbon atoms and may be identical to or different from one another.

Examples of the aliphatic hydrocarbon groups having 1 to 20 carbon atoms which are represented by R⁵¹², R⁵¹³, and R⁵¹⁴ are the same as the groups described above as examples of the aliphatic hydrocarbon group having 1 to 20 carbon atoms which is represented by R⁵¹¹.

Among these, it is preferable that R⁵¹², R⁵¹³, and R⁵¹⁴ be alkylene groups having 2 to 10 carbon atoms. It is also preferable that R⁵¹², R⁵¹³, and R⁵¹⁴ be the same groups in consideration of synthesis efficiency, etc.

In Formula (3a₁), M⁵¹¹ and M⁵¹² each independently represent an alkali metal ion and may be identical to or different from one another.

Examples of the alkali metal ion include one or more selected from the group consisting of Li, Na, and K. It is preferable that M⁵¹¹ and M⁵¹² be the same alkali metal ions in consideration of synthesis efficiency, etc.

Formula (3a₂) is the group represented by the following formula.

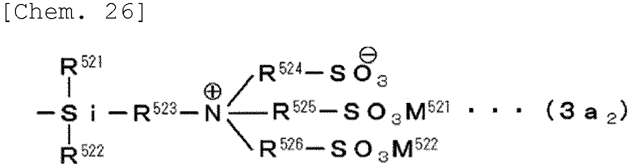

In Formula (3a₂), R⁵²¹ and R⁵²² each independently represent a monovalent aliphatic hydrocarbon group having 1 to 20 carbon atoms or a monovalent aromatic hydrocarbon group having 6 to 30 carbon atoms. These groups may include one or more selected from -O- (ether group), -S- (thioether group), -NH- (amine group), -COO- (ester group), -NHCOO-(urethane group), -NHCONH- (urea group), -NHCSNH- (thiourea group), -NHCO- (amide group), a nitrogen-oxygen bond, a nitrogen-nitrogen bond, an oxygen-phosphorus bond, a sulfur-phosphorus bond, and the like and may have one or more substituents.

The aliphatic hydrocarbon group is, for example, a linear or branched divalent aliphatic hydrocarbon group having 1 to 20 carbon atoms and is preferably a linear or branched aliphatic hydrocarbon group having 4 to 13 carbon atoms. Specific examples thereof include an alkyl group, an alkenyl group, and an alkynyl group.

Preferable examples of the alkyl group include alkyl groups having 4 to 13 carbon atoms, such as methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, and decyl.

Preferable examples of the alkenyl group include alkenyl groups having 2 to 10 carbon atoms, such as ethenyl, 1-propenyl, 2-propenyl, 2-methyl-1-propenyl, 1-butenyl, 2-butenyl, 3-butenyl, 3-methyl-2-butenyl, 1-pentenyl, 4-methyl-3-pentenyl, 1-hexenyl, 1-heptenyl, and 1-octenyl.

Preferable examples of the alkynyl group include alkynyl groups having 2 to 10 carbon atoms, such as ethynyl, 1-propynyl, 2-propynyl, 1-butynyl, 1-pentynyl, 2-pentynyl, 1-hexynyl, 2-hexynyl, 1-heptynyl, and 1-octynyl.

The aromatic hydrocarbon group is, for example, an aromatic hydrocarbon group having 6 to 30 carbon atoms and is preferably an aryl group having 6 to 14 carbon atoms. Examples thereof include phenyl, naphthyl, anthryl, phenanthryl, acenaphthylenyl, biphenylyl, and indenyl. Among these, phenyl, naphthyl, and the like are preferable. The aryl group may be partially saturated. Examples of the partially saturated aryl group include dihydroindenyl.

The substituent that may be included in the monovalent aliphatic hydrocarbon group having 1 to 20 carbon atoms or the monovalent aromatic hydrocarbon group having 6 to 30 carbon atoms may be one or more substituents present at the positions at which the hydrocarbon group may have substituents. Examples of the above substituent include one or more selected from the group consisting of a halogen atom, a nitro group, a carboxyl group, a hydroxyl group, a silyl group, an alkoxy group having 1 to 3 carbon atoms, a heterocyclic group, an amino group, a thiol group, an acyl group, a phosphate group, and the like.

In Formula (3a₂), R⁵²³ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms.

R⁵²⁴, R⁵²⁵, and R⁵²⁶ each independently represent a divalent aliphatic hydrocarbon group having 1 to 20 carbon atoms and may be identical to or different from one another. M⁵²¹ and M⁵²² each independently represent an alkali metal ion and may be identical to or different from one another.)

Examples of the group represented by R⁵²³ are the same as the groups described above as examples of the group represented by R⁵¹¹.

Examples of the groups represented by R⁵²⁴, R⁵²⁵, and R⁵²⁶ are the same as the groups described above as examples of the groups represented by R⁵¹², R⁵¹³, and R⁵¹⁴. It is preferable that R⁵²⁴, R⁵²⁵, and R⁵²⁶ be the same groups in consideration of synthesis efficiency, etc.

Examples of the alkali metal ions represented by M⁵²¹ and M⁵²² are the same as the alkali metal ions described above as examples of the alkali metal ions represented by M⁵¹¹ and M⁵¹². It is preferable that M⁵²¹ and M⁵²² be the same alkali metal ions in consideration of synthesis efficiency, etc.

Formula (3a₃) is the group represented by the following formula.

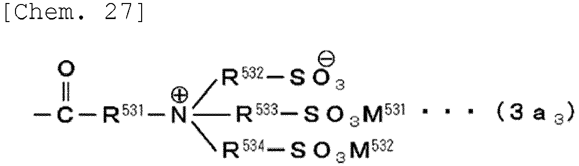

In Formula (3a₃), R⁵³¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms.

In Formula (3a₃), R⁵³², R⁵³³, and R⁵³⁴ each independently represent a divalent aliphatic hydrocarbon group having 1 to 20 carbon atoms and may be identical to or different from one another.

In Formula (3a₃), M⁵³¹ and M⁵³² each independently represent an alkali metal ion and may be identical to or different from one another.

Examples of the group represented by R⁵³¹ are the same as the groups described above as examples of the group represented by R⁵¹¹.

Examples of the groups represented by R⁵³², R⁵³³, and R⁵³⁴ are the same as the groups described above as examples of the groups represented by R⁵¹², R⁵¹³, and R⁵¹⁴. It is preferable that R⁵³², R⁵³³, and R⁵³⁴ be the same groups in consideration of synthesis efficiency, etc.

Examples of the alkali metal ions represented by M⁵³¹ and M⁵³² are the same as the alkali metal ions described above as examples of the alkali metal ions represented by M⁵¹¹ and M⁵¹². It is preferable that M⁵³¹ and M⁵³² be the same alkali metal ions in consideration of synthesis efficiency, etc.

In Formula (3), A⁴⁰² is any of a hydrogen atom and the groups represented by Formulae (3b₁), (3b₂), and (3b₃).

Formula (3b₁) is the group represented by the following formula.

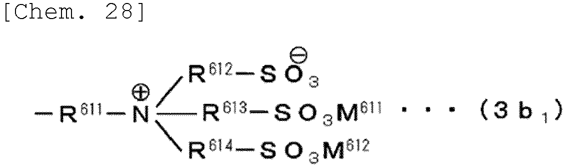

In Formula (3b₁), R⁶¹¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms.

In Formula (3b₁), R⁶¹², R⁶¹³, and R⁶¹⁴ each independently represent a divalent aliphatic hydrocarbon group having 1 to 20 carbon atoms and may be identical to or different from one another.

In Formula (3b₁), M⁶¹¹ and M⁶¹² each independently represent an alkali metal ion and may be identical to or different from one another.

Examples of the group represented by R⁶¹¹ are the same as the groups described above as examples of the group represented by R⁵¹¹.

Examples of the groups represented by R⁶¹², R⁶¹³, and R⁶¹⁴ are the same as the groups described above as examples of the groups represented by R⁵¹², R⁵¹³, and R⁵¹⁴. It is preferable that R⁶¹², R⁶¹³, and R⁶¹⁴ be the same groups in consideration of synthesis efficiency, etc.

Examples of the alkali metal ions represented by M⁶¹¹ and M⁶¹² are the same as the alkali metal ions described above as examples of the alkali metal ions represented by M⁵¹¹ and M⁵¹². It is preferable that M⁶¹¹ and M⁶¹² be the same alkali metal ions in consideration of synthesis efficiency, etc.

Formula (3b₂) is the group represented by the following formula.

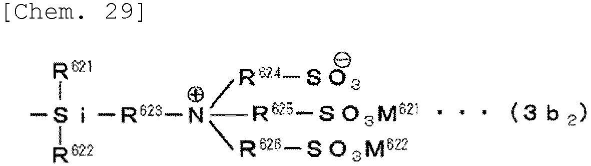

In Formula (3b₂), R⁶²¹ and R⁶²² each independently represent a monovalent aliphatic hydrocarbon group having 1 to 20 carbon atoms or a divalent aromatic hydrocarbon group having 6 to 30 carbon atoms.

In Formula (3b₂), R⁶²³ represents -CH₂-, -CH₂-CH₂-, - CH(CH₃)CH₂-, -CH₂CH(CH₃)-, a divalent aliphatic hydrocarbon group having 4 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms.

In Formula (3b₂), R⁶²⁴, R⁶²⁵, and R⁶²⁶ each independently represent a divalent aliphatic hydrocarbon group having 1 to 20 carbon atoms and may be identical to or different from one another.

In Formula (3b₂), M⁶²¹ and M⁶²² each independently represent an alkali metal ion and may be identical to or different from one another.

Examples of the groups represented by R⁶²¹ and R⁶²² are the same as the groups described above as examples of the groups represented by R⁵²², R⁵²², and R⁵¹⁴.

Examples of the group represented by R⁶²³ are the same as the groups described above as examples of the group represented by R⁵¹¹.

Examples of the groups represented by R⁶²⁴, R⁶²⁵, and R⁶²⁶ are the same as the groups described above as examples of the groups represented by R⁵¹², R⁵¹³, and R⁵¹⁴. It is preferable that R⁶²⁴, R⁶²⁵, and R⁶²⁶ be the same groups in consideration of synthesis efficiency, etc.

Examples of the alkali metal ions represented by M⁶²¹ and M⁶²² are the same as the alkali metal ions described above as examples of the alkali metal ions represented by M⁵¹¹ and M⁵¹². It is preferable that M⁶²¹ and M⁶²² be the same alkali metal ions in consideration of synthesis efficiency, etc.

Formula (3b₃) is the group represented by the following formula.

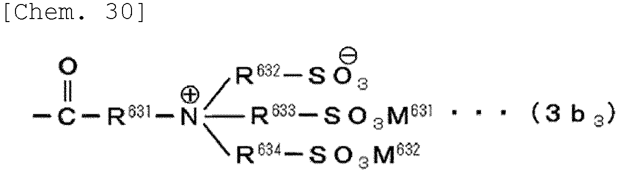

In Formula (3b₃), R⁶³¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms.

In Formula (3b₃), R⁶³², R⁶³³, and R⁶³⁴ each independently represent a divalent aliphatic hydrocarbon group having 1 to 20 carbon atoms and may be identical to or different from one another.

In Formula (3b₃), M⁶³¹ and M⁶³² each independently represent an alkali metal ion and may be identical to or different from one another.

Examples of the group represented by R⁶³¹ are the same as the groups described above as examples of the group represented by R⁵¹¹.

Examples of the groups represented by R⁶³², R⁶³³, and R⁶³⁴ are the same as the groups described above as examples of the groups represented by R⁵¹², R⁵¹³, and R⁵¹⁴. It is preferable that R⁶³², R⁶³³, and R⁶³⁴ be the same groups in consideration of synthesis efficiency, etc.

Examples of the alkali metal ions represented by M⁶³¹ and M⁶³² are the same as the alkali metal ions described above as examples of the alkali metal ions represented by M⁵¹¹ and M⁵¹². It is preferable that M⁶³¹ and M⁶³² be the same alkali metal ions in consideration of synthesis efficiency, etc.

### <Combination of R⁴¹¹, R⁴²¹, R⁴³¹, R⁴⁴¹, w, x, y, z, A⁴⁰¹, and A402>

The combination of R⁴¹¹, R⁴²¹, R⁴³¹, R⁴⁴¹, w, x, y, z, A⁴⁰¹, and A⁴⁰² can be changed appropriately within the above-described range. Among these, for example, the following combinations are preferable.
(i) w = x = y = z = 0, A⁴⁰¹ = Formula (3a₁), A⁴⁰² = Formula (3b₁), R⁵¹¹ = R⁶¹¹ = alkylene group having 1 to 20 carbon atoms, R⁵¹² = R⁵¹³ = R⁵¹⁴ = R⁶¹² = R⁶¹³ = R⁶¹⁴ = alkylene group having 1 to 20 carbon atoms, and M⁵¹¹ = M⁵¹² = M⁶¹¹ = M⁶¹² = Na
(ii) w = x = y = z = 0, A⁴⁰¹ = Formula (3a₁), A⁴⁰² = Formula (3b₁), R⁵¹¹ = R⁶¹¹ = phenylalkylene group having 7 to 20 carbon atoms, R⁵¹² = R⁵¹³ = R⁵¹⁴ = R⁶¹² = R⁶¹³ = R⁶¹⁴ = alkylene group having 1 to 20 carbon atoms, and M⁵¹¹ = M⁵¹² = M⁶¹¹ = M⁶¹² = Na
(iii) w = x = y = z = 0, A⁴⁰¹ = Formula (3a₃), A⁴⁰² = Formula (3b₃), R⁵³¹ = R⁶³¹ = alkylene group having 1 to 20 carbon atoms, R⁵³² = R⁵³³ = R⁵³⁴ = R⁶³² = R⁶³³ = R⁶³⁴ = alkylene group having 1 to 20 carbon atoms, and M⁵³¹ = M⁵³² = M⁶³¹ = M⁶³² = Na
(iv) w = x = y = z = 0, A⁴⁰¹ = Formula (3a₁), A⁴⁰² = H, R⁵¹¹ = alkylene group having 1 to 20 carbon atoms, R⁵¹² = R⁵¹³ = R⁵¹⁴ = alkylene group having 1 to 20 carbon atoms, and M⁵¹¹ = M⁵¹² = Na
(v) w = x = y = z = 0, A⁴⁰¹ = Formula (3a₁), A⁴⁰² = H, R⁵¹¹ = phenylalkylene group having 7 to 20 carbon atoms, R⁵¹² = R⁵¹³ = R⁵¹⁴ = alkylene group having 1 to 20 carbon atoms, and M⁵¹¹ = M⁵¹² = Na
(vi) w = x = y = z = 0, A⁴⁰¹ = Formula (3a₂), A⁴⁰² = H, R⁵²¹ = R⁵²² = alkyl group having 1 to 20 carbon atoms, R⁵²³ = alkylene group having 1 to 20 carbon atoms, R⁵²⁴ = R⁵²⁵ = R⁵²⁶ = alkylene group having 1 to 20 carbon atoms, and M⁵¹¹ = M⁵¹² = Na
(vii) w = x = y = z = 0, A⁴⁰¹ = Formula (3a₃), A⁴⁰² = H, R⁵³¹ = alkylene group having 1 to 20 carbon atoms, R⁵³² = R⁵³³ = R⁵³⁴ = alkylene group having 1 to 20 carbon atoms, and M⁵³¹ = M⁵³² = Na
(viii) w = x = y = 0, z = 1, A⁴⁰¹ = Formula (3a₁), A⁴⁰² = Formula (3b₁), R⁵¹¹ = R⁶¹¹ = alkylene group having 1 to 20 carbon atoms, R⁵¹² = R⁵¹³ = R⁵¹⁴ = R⁶¹² = R⁶¹³ = R⁶¹⁴ = alkylene group having 1 to 20 carbon atoms, M⁵¹¹ = M⁵¹² = M⁶¹¹ = M⁶¹² = Na, R⁴⁴¹ = the group represented by the following formula (where * denotes the position at which the group is bonded to the phthalocyanine skeleton) 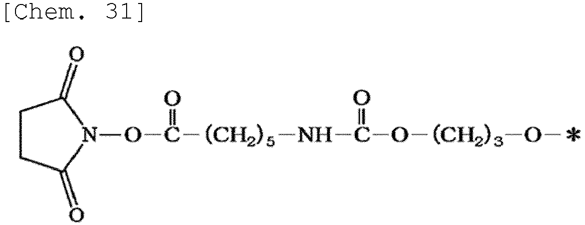
(ix) w = x = y = 0, z = 1, A⁴⁰¹ = Formula (3a₁), A⁴⁰² = Formula (3b₁), R⁵¹¹ = R⁶¹¹ = phenylalkylene group having 7 to 20 carbon atoms, R⁵¹² = R⁵¹³ = R⁵¹⁴ = R⁶¹² = R⁶¹³ = R⁶¹⁴ = alkylene group having 1 to 20 carbon atoms, M⁵¹¹ = M⁵¹² = M⁶¹¹ = M⁶¹² = Na, R⁴⁴¹ = the group represented by the following formula (where * denotes the position at which the group is bonded to the phthalocyanine skeleton) 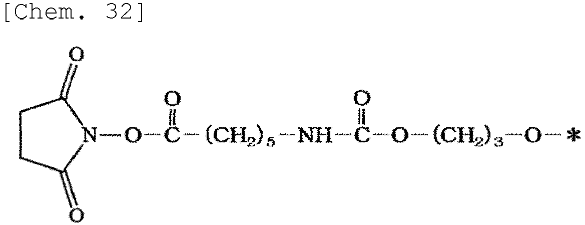
(x) w = x = y = 0, z = 1, A⁴⁰¹ = Formula (3a₂), A⁴⁰² = H, R⁵²¹ = R⁵²² = alkyl group having 1 to 20 carbon atoms, R⁵²³ = alkylene group having 1 to 20 carbon atoms, R⁵²⁴ = R⁵²⁵ = R⁵²⁶ = alkylene group having 1 to 20 carbon atoms, M⁵¹¹ = M⁵¹² = Na, R⁴⁴¹ = the group represented by the following formula (where * denotes the position at which the group is bonded to the phthalocyanine skeleton) 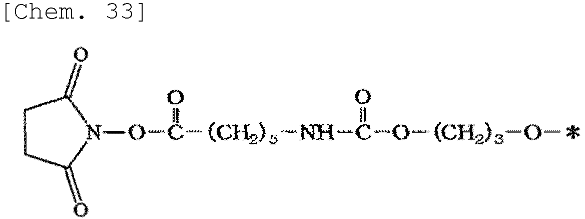
(xi) w = x = y = 0, z = 1, A⁴⁰¹ = Formula (3a₃), A⁴⁰² = Formula (3b₃), R⁵³¹ = R⁶³¹ = alkylene group having 1 to 20 carbon atoms, R⁵³² = R⁵³³ = R⁵³⁴ = R⁶³² = R⁶³³ = R⁶³⁴ = alkylene group having 1 to 20 carbon atoms, M⁵³¹ = M⁵³² = M⁶³¹ = M⁶³² = Na, R⁴⁴¹ = the group represented by the following formula (where * denotes the position at which the group is bonded to the phthalocyanine skeleton) 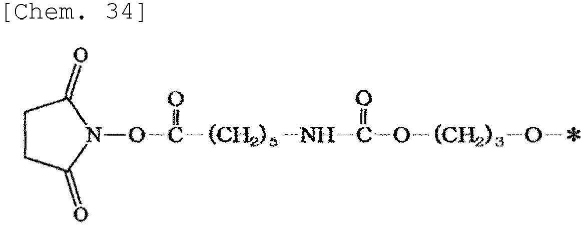
(xii) w = x = y = 0, z = 1, A⁴⁰¹ = Formula (3a₁), A⁴⁰² = H, R⁵¹¹ = alkylene group having 1 to 20 carbon atoms, R⁵¹² = R⁵¹³ = R⁵¹⁴ = alkylene group having 1 to 20 carbon atoms, M⁵¹¹ = M⁵¹² = Na, R⁴⁴¹ = the group represented by the following formula (where * denotes the position at which the group is bonded to the phthalocyanine skeleton) 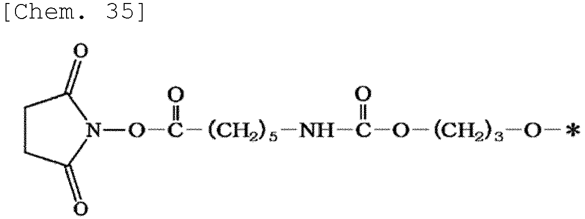
(xiii) w = x = y = 0, z = 1, A⁴⁰¹ = Formula (3a₁), A⁴⁰² = H, R⁵¹¹ = phenylalkylene group having 7 to 20 carbon atoms, R⁵¹² = R⁵¹³ = R⁵¹⁴ = alkylene group having 1 to 20 carbon atoms, M⁵¹¹ = M⁵¹² = Na, R⁴⁴¹ = the group represented by the following formula (where * denotes the position at which the group is bonded to the phthalocyanine skeleton) 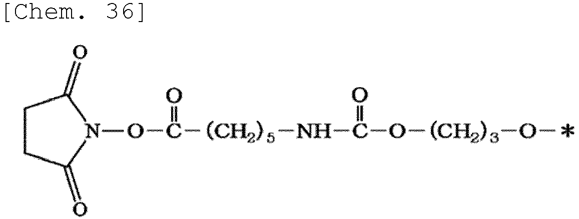
(xiv) w = x = y = 0, z = 1, A⁴⁰¹ = Formula (3a₃), A⁴⁰² = H, R⁵³¹ = alkylene group having 1 to 20 carbon atoms, R⁵³² = R⁵³³ = R⁵³⁴ = alkylene group having 1 to 20 carbon atoms, M⁵³¹ = M⁵³² = Na, R⁴⁴¹ = the group represented by the following formula (where * denotes the position at which the group is bonded to the phthalocyanine skeleton)

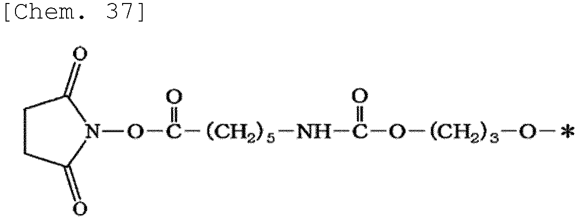

The results of the quantum chemical calculation conducted by the inventors of the present invention confirmed that the likelihood of protonation of a compound that includes a silicon phthalocyanine skeleton and, for example, an alkoxy group, a siloxy group, an oxycarbonyl group, or a phenoxy group which is introduced to the skeleton as an axial ligand decreases in the order of alkoxy group > siloxy group > oxycarbonyl group > phenoxy group.

The inventors of the present invention prepared compounds that included a silicon phthalocyanine skeleton and an alkoxy group, a siloxy group, an oxycarbonyl group, or a phenoxy group introduced to the silicon phthalocyanine skeleton as an axial ligand, blew an inert gas into aqueous solutions of the compounds in the presence of an electron donor (water) by bubbling, then irradiated the aqueous solutions with near-ultraviolet light, and conducted an analysis with a high-performance liquid chromatography (HPLC). As a result, the dissociation of the axial ligands was confirmed. Furthermore, it was confirmed that the dissociation of the axial ligands also occurs even in the case where the above compounds were irradiated with X-rays.

### [Method for Producing Compound]

The method for producing the compound represented by Formula (3) is not limited; the compound (3) can be produced in accordance with, for example, PTL 1 or 2 or NPL 1 or 2 listed above.

For example, in the case where the compound (3) includes the group represented by Formula (3a₁), (3a₃), (3b₁), or (3b₃) as an axial ligand, the compound (3) can be produced under the reaction scheme 1 below.

For example, in the case where the compound (3) includes the groups represented by Formula (3a₂) as axial ligands, the compound (3) can be produced under the reaction scheme 2 below.

For example, in the case where the compound (3) includes only one group represented by Formula (3a₂) as an axial ligand, the compound (3) can be produced under the reaction scheme 3 below.

An appropriate solvent, reaction catalyst, reaction accelerator, or the like may be used as needed in any steps of each reaction scheme.

A temperature control such as heating, atmosphere adjustment, pH adjustment, adjustment of reaction time, stirring, and the like may be performed as needed in any steps of each reaction scheme.

The resulting compound may be subjected to separation, cleaning, purification, or the like by publicly known means.

### <Reaction Scheme 1>

A reaction scheme for a compound represented by Formula (3) which satisfies, for example, the following conditions:
all of w, x, y, and z are zero;
A⁴⁰¹ is a group represented by Formula (3a₁) or (3a₃);
R⁵¹¹ is a group represented by -R⁵¹⁹-CH₂- (where R⁵¹⁹ represents a divalent aliphatic group having 1 to 19 carbon atoms or a divalent aromatic group having 6 to 30 carbon atoms);
R⁵¹³ is a group represented by -CO-R⁵³⁹- (where R⁵³⁹ represents a divalent aliphatic group having 1 to 19 carbon atoms);
all of R⁵¹², R⁵¹³, R⁵¹⁴, R⁵³², R⁵³³, and R⁵³⁴ are - (CH₂) ₃-;
all of M⁵¹¹, M⁵¹², M⁵³¹, and M⁵³² are Na; and
A⁴⁰² is a group represented by Formula (3a₁) or (3a₃) and is the same as the group represented by A⁴⁰¹;
is as follows.

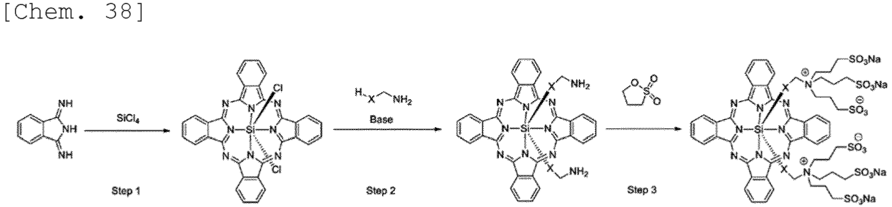

The step 1 of the scheme 1 is a step of causing 1,3-diiminoisoindoline and silicon tetrachloride to react with each other to synthesize silicon phthalocyanine dichloride.

In the step 1, the reaction is preferably conducted in a solvent. Examples of the solvent include, but are not limited to, one or more selected from the group consisting of basic solvents (e.g., pyridine, quinoline, triethylamine, and tributylamine); aprotic polar solvents (e.g., tetrahydrofuran, diethyl ether, dimethylformamide, dimethylacetamide, N-methylpyrrolidone, dimethyl sulfoxide, dimethyl imidazolidinone, N,N'-dimethylethyleneurea, diethyl ether, dibutyl ether, acetone, methyl ethyl ketone, methyl isobutyl ketone, cyclohexanone, methyl acetate, ethyl acetate, butyl acetate, γ-butyrolactone, dimethyl carbonate, acetonitrile, and nitrobenzene); and nonpolar solvents (e.g., benzene, xylene, toluene, and naphthalene). Among these, basic solvents, such as quinoline and tributylamine, are preferable. It is also preferable to use a mixed solvent of a basic solvent, such as quinoline or tributylamine, and a nonpolar solvent, such as naphthalene or toluene.

In the step 1, the reaction is preferably conducted at 100°C or more, for example, at reflux. The reaction time is commonly, but not limited to, 0.1 to 24 hours.

The step 2 of the scheme 1 is a step of causing the silicon phthalocyanine dichloride prepared in the step 1 to react with a compound that forms an axial ligand in the presence of a base.

Examples of the compound that forms an axial ligand include a hydroxyl group-containing compound and a carboxyl group compound.

Examples of the base include one or more selected from the group consisting of sodium hydride, lithiumdiisopropylamide, potassium carbonate, sodium carbonate, triethylamine, diisopropylethylamine, and tributylamine. Among these, one or more selected from the group consisting of sodium hydride, potassium carbonate, sodium carbonate, and the like are preferable.

In the step 2, the reaction is preferably conducted in a solvent. Examples of the solvent include nonpolar solvents (e.g., benzene, toluene, xylene, and naphthalene).

In the step 1, the reaction is preferably conducted by performing heating at 50°C or more. The reaction time is commonly, but not limited to, 0.1 to 24 hours.

The step 3 of the scheme 1 is a step of causing the reaction product prepared in the step 2 to react with a sultone in a protic polar solvent in the presence of a tertiary amine to synthesize the compound according to the present invention. Optionally, cleaning and purification may be performed as needed. Cation exchange may also be performed as needed.

The sultone is not limited to the 1,3-propanesultone described above as an example; for example, one or more selected from the group consisting of 1,4-butanesultone, 1,5-pentanesultone, 1,6-hexanesultone, 1,7-heptanesultone, and 1,8-octanesultone may be used.

Examples of the tertiary amine include one or more selected from the group consisting of diisopropylethylamine, triethylamine, and tributylamine.

Examples of the protic polar solvent include alcohol solvents, such as methanol and ethanol.

The method for performing the optional cleaning and purification is not limited; publicly known methods may be used. It is preferable to perform the cleaning and purification under neutral conditions, because the product may become decomposed under acidic conditions. In particular, it is preferable to perform the cleaning and purification with a reverse-phase column using a neutral solution (buffer solution) and an aprotic positively polarized solvent as eluents.

The method for performing the optional cation exchange is not limited; publicly known methods may be used. In particular, it is preferable to perform the cation exchange using an ion-exchange resin (cation-exchange resin).

### <Scheme 2>

A reaction scheme for a compound represented by Formula (3) which satisfies, for example, the following conditions:
all of w, x, y, and z are zero;
A⁴⁰¹ is a group represented by Formula (3a₂) ;
R⁵²¹ and R⁵²² are methyl groups;
R⁵²³ is a group represented by - (CH₂)₃-;
all of R⁵²⁴, R⁵²⁵, and R⁵²⁶ are - (CH₂) ₃-;
all of M⁵²¹ and M⁵²² are Na; and
A⁴⁰² is a group represented by Formula (3a₂) and is the same as the group represented by A⁴⁰¹;
is as follows.

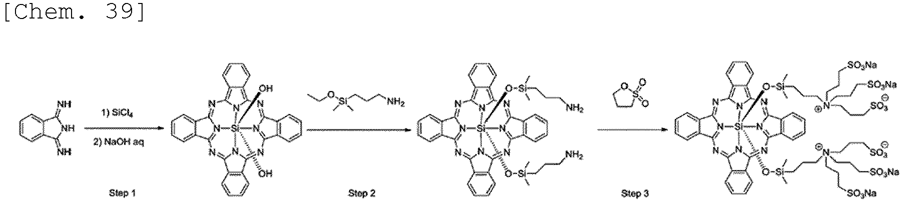

The step 1 of the scheme 2 is a step of causing 1,3-diiminoisoindoline and silicon tetrachloride to react with each other to synthesize silicon phthalocyanine dichloride and further causing the silicon phthalocyanine dichloride to react with a hydroxide to synthesize silicon phthalocyanine dihydroxide.

The synthesis of silicon phthalocyanine dichloride can be conducted as in the step 1 of the scheme 1.

The hydroxide caused to react with silicon phthalocyanine dichloride is not limited to the aqueous sodium hydroxide solution described above as an example; for example, aqueous solutions of various alkali metal hydroxides may be used.

The step 2 of the scheme 2 is a step of causing the silicon phthalocyanine dihydroxide prepared in the step 1 to react with an alkoxysilane compound that forms an axial ligand in the presence of a base.

The alkoxysilane compound that forms an axial ligand is not limited to the 3-aminopropyldimethylethoxysilane described above as an example; any alkoxysilane compounds including an amino group may be used.

Examples of the base include basic solvents (e.g., pyridine, quinoline, triethylamine, and tributylamine).

In the step 2, the reaction may be conducted by performing heating as needed. The reaction time is commonly, but not limited to, 0.1 to 24 hours.

The step 3 of the scheme 2 is a step of causing the reaction product prepared in the step 2 to react with a sultone in a protic polar solvent in the presence of a tertiary amine to synthesize the compound according to the present invention. Optionally, cleaning and purification may be performed as needed. Cation exchange may also be performed as needed.

The step 3 of the scheme 2 can be conducted as in the step 3 of the scheme 1.

### <Reaction Scheme 3>

The reaction scheme 3 is a scheme under which a compound having one axial ligand can be produced from the compound produced under the reaction scheme 1 or 2 which has two axial ligands. The reaction scheme 3 includes a step of mixing the compound having two axial ligands with an acid.

In the reaction scheme for a compound represented by Formula (3) which satisfies, for example, the following conditions:
all of w, x, y, and z are zero;
A⁴⁰¹ is a group represented by Formula (3a₂);
R⁵²¹ and R⁵²² are methyl groups;
R⁵²³ is a group represented by - (CH₂) ₃-;
all of R⁵²⁴, R⁵²⁵, and R⁵²⁶ are - (CH₂)₃-;
all of M⁵²¹ and M⁵²² are Na; and
A⁴⁰² is H;
the following step is conducted subsequent to the production of the compound in the reaction scheme 2.

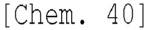

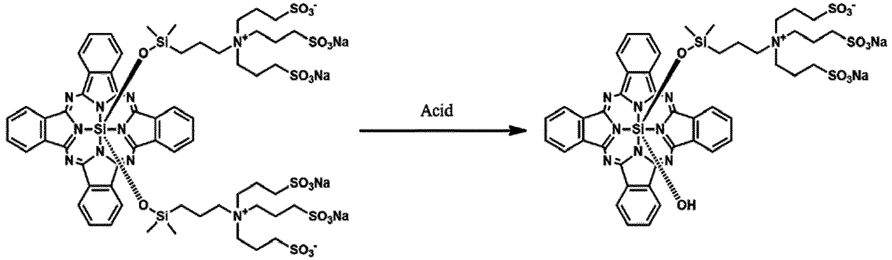

In the scheme 3, optionally, cleaning and purification may be performed as needed. Optionally, cation exchange may be performed as needed.

In the scheme 3, as an acid, any inorganic or organic acid may be used. In the present invention, it is preferable to use an acidic buffer solution having a pH of 1 to 5 in order to maintain the pH within a predetermined range.

The cleaning, purification, and cation exchange can be performed by cleaning and purification means and cation exchange means similar to those used in the step 3 of the scheme 1.

### [Application of Compound]

The compound according to the second embodiment of the present invention can be used as a chemical for disease or disease state selected from the group consisting of vascular disease, cancer, bacterial infection, antibiotic-resistant wound infection, actinic keratosis, acne vulgaris, and psoriasis or as a compound that serves as a raw material for such a chemical.

Examples of the vascular disease include wet age-related macular degeneration. The cancer is selected from the group consisting of breast cancer, colorectal cancer, esophageal cancer, bronchial cancer, Barrett's esophagus with high-grade dysplasia, lung cancer, prostatic cancer, cervical cancer, ovarian cancer, stomach cancer, pancreas cancer, liver cancer, bladder cancer, brain tumor, head and neck cancer, neuroendocrine carcinoma, skin cancer, and combinations thereof. In particular, the above-described compound can be used as a chemical for photoimmunotherapy of unresectable, locally-advanced cancer, locally recurrent cancer, or the like or as a compound that serves as a raw material for such a chemical.

### EXAMPLES

Further details of the present invention are described with reference to Production Examples, Examples, and Comparative Examples below. Note that Examples below are merely an aspect of the present invention and do not limit the present invention.

### [Production Example 1]

### <Synthesis of Silicon Phthalocyanine Dihydroxide (pc1)>

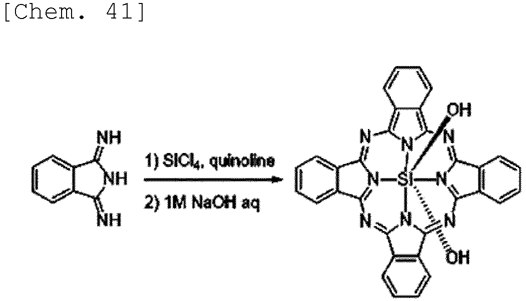

Silicon tetrachloride (300 mg; 1.77 mmol) and 1,3-diiminoisoindoline (176 mg; 1.21 mmol) were dissolved in quinoline (2 ml). The resulting mixture was caused to reflux for 2 hours in an argon atmosphere.

After the mixture had been cooled to room temperature, a 1-M aqueous sodium hydroxide solution (2 ml) was added to the mixture. Subsequently, the mixture was caused to reflux for 1 hour and then filtered to obtain a reaction product. The reaction product was cleaned with methanol and then vacuum-dried. Hereby, 111 mg (0.194 mmol) of a reaction product (pc1) was prepared.

MS (MALDI⁺) : m/z calcd for C₃₂H₁₉N₈O₂Si: 575.1: [M+H]⁺; found: 574.7.

### [Production Example 2]

### <Synthesis of Bis(3-Aminopropyldimethylsilyl Oxide) Silicon Phthalocyanine (pc2)>

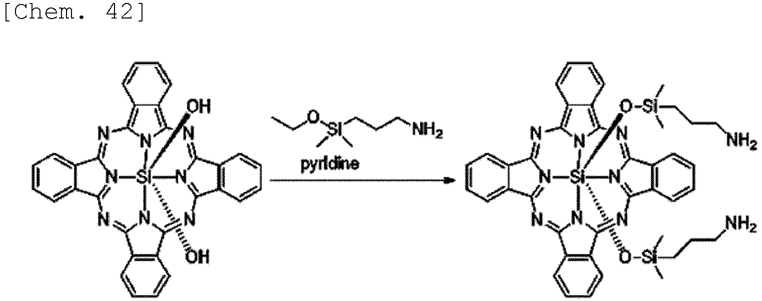

The pc1 (50 mg; 0.087 mmol) and 3-aminopropyldimethylethoxysilane (140 mg; 0.87 mmol) were dissolved in pyridine (40 ml). The resulting mixture was caused to reflux for 6 hours in an argon atmosphere.

Subsequently, concentration was performed at 35°C or less by rotary evaporation. The residue was diluted and then filtered. Subsequent to the filtration, cleaning was performed with a water-ethanol liquid mixture (water 2: ethanol 1). Then, vacuum drying was performed. Hereby, 47 mg (0.0583 mmol; yield: 43%) of a reaction product (pc2) was prepared.
¹H NMR(400 MHz, CDCl₃): δ -2.86(s, 12H), -2.34 to -2.27 (m, 4H), -1.28 to -1.19 (m, 4H), 1.18 (t, J = 7.2 Hz, 4H), 8.35 (dd, J = 5.7, 2.9 Hz, 8H), 9.65 (dd, J = 5.7, 2.9 Hz, 8H) ;
HRMS (ESI⁺) : m/z calcd for C₄₂H₄₅N₁₀O₂Si₃Na: 827.2849: [M+Na]⁺; found: 827.2848.

### [Reference Example 1]

### <Synthesis of Bis{3-[Tris(3-Sulfopropyl)]Ammoniopropyldimethylsilyl Oxide} Silicon Phthalocyanine (Compound 1)>

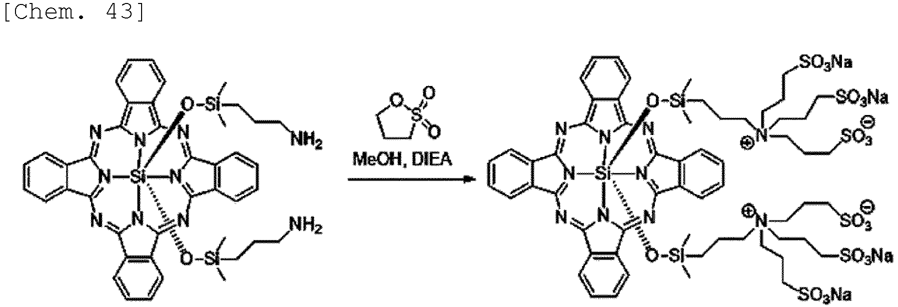

The pc2 (40 mg, 0.050 mmol), 1,3-propanesultone (72.8 mg, 0.60 mmol), and N,N-diisopropylethylamine (DIEA, 84.7 mg, 0.66 mmol) were dissolved in methanol (2 ml). The resulting mixture was stirred for 48 hours at 50°C in an argon atmosphere.

The product was cleaned and purified using an eluent A (water, 0.1 M triethylammonium acetic acid salt (TEAA)) and an eluent B (acetonitrile 99%, water 1%) (A/B: 80/20 to 50/50 for 15 minutes and 50/50 to 0/100 for 5 minutes) with an HPLC system (produced by Shimadzu Corporation) equipped with a reverse-phase column Inertsil ODS-3 (10 mm × 250 mm) (produced by GL Sciences).

The product was then subjected to cation exchange using Sep-Pak C18 cartridge (produced by Waters) and a cation-exchange resin (Amberlite IR120B Na produced by Organo Corporation). Hereby, 16.3 mg (0.010 mmol, yield as soda salt: 20%) of a compound 1 was prepared.

¹H NMR (400 MHz, CD₃OH) : δ -2.79 (s, 12H), -2.15 (t, J = 8.1 Hz, 4H), -0.87 to -0.97 (m, 4H), 1.76 to 1.66 (m, 12H), 2.02 (t, J = 8.1 Hz, 4H), 2.81 to 2.72 (m, 24H);
HRMS (ESI⁺) : m/z calcd for C₆₀H₇₆N₁₀Na₅O₂₀S₆Si₃: 1647.2358: [M+Na]⁺; found: 1647.2358.

### [Production Example 3]

### <Synthesis of Silicon Phthalocyanine Dichloride (pc3)>

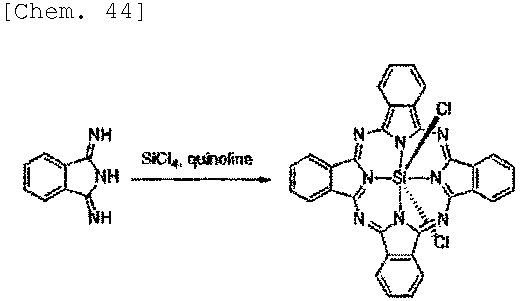

1,3-Diiminoisoindoline (176 mg, 1.21 mmol) and silicon tetrachloride (300 mg, 1.8 mmol) were dissolved in quinoline (2 ml). The resulting mixture was caused to reflux for 2 hours in an argon atmosphere.

After the mixture had been cooled to room temperature, methanol was added to the mixture. The resulting precipitate was filtered to obtain a reaction product. The reaction product was cleaned with methanol and then vacuum-dried. Hereby, 123 mg of a reaction product (pc3) was prepared.

### [Production Examples 4 to 6]

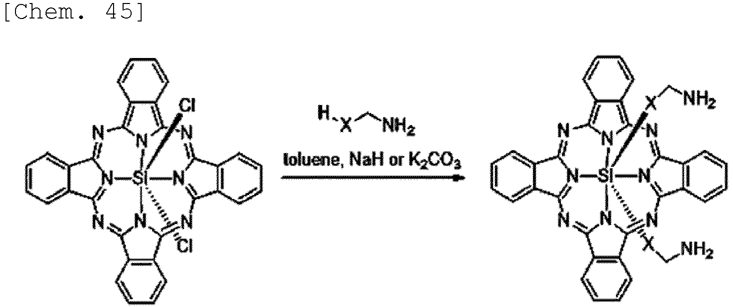

### [Production Example 4]

### <Synthesis of Bis(4-Aminobutyloxide) Silicon Phthalocyanine (pc4)>

The crude pc3 (50 mg, about 0.082 mmol), 4-aminobutanol (73 mg, 0.82 mmol), and sodium hydride (39 mg, 1.6 mmol) were dissolved in toluene (40 ml). The resulting mixture was caused to reflux for 8 hours in an argon atmosphere.

After the mixture had been cooled to room temperature, the mixture was concentrated by rotary evaporation at 35°C or less. The residue was cleaned with a water-ethanol liquid mixture (water 2: ethanol 1) and then vacuum-dried. Hereby, 31 mg (0.043 mmol; yield: 52%) of a reaction product (pc4) was prepared.

¹H NMR (400 MHz, CDCl₃): δ -2.10 (t, J = 6.1 Hz, 4H), - 1.64 (tt, J = 6.1, 7.2 Hz, 4H), -1.25 (tt, J = 7.2, 7.2 Hz, 4H), 0.94 (t, J =7.2 Hz, 4H), 8.34 (dd, J = 2.9, 5.6 Hz, 8H), 9.64 (dd, J = 2.9, 5.6 Hz, 8H) . HRMS (ESI⁺) m/z: calcd for C₄₀H₃₈N₁₀O₂Si: 359.1470 [M+2H]²⁺; found: 359.1469.

### [Production Example 5]

### <Synthesis of Bis[(6-Aminohexanoyl)Oxide] Silicon Phthalocyanine (pc5)>

The crude pc3 (300 mg, about 0.49 mmol), 6-aminohexanoic acid (640 mg, 4.9 mmol), and potassium carbonate (K₂CO₃; 200 mg, 1.5 mmol) were dissolved in toluene (20 ml). The resulting mixture was caused to reflux for 8 hours in an argon atmosphere.

After the mixture had been cooled to room temperature, the mixture was concentrated at 35°C or less. Dichloromethane was added to the residue to form a suspension. The resulting precipitate was filtered to obtain a reaction product (pc5). The reaction product (pc5) was cleaned with dichloromethane and then vacuum-dried. Hereby, 76 mg (0.094 mmol, 14%)) of a reaction product (pc5) was prepared.

¹H NMR (400 MHz, CDCl₃): δ -0.92 to -0.89 (m, 4H), -0.79 to -0.71 (m, 4H), -0.64 to -0.62 (m, 4H), 0.15 to 0.23 (m, 4H), 1.94 (t, J = 6.9 Hz, 4H), 8.39 (dd, J = 2.7, 5.7 Hz, 8H), 9.70 (dd, J = 2.7, 5.7 Hz, 8H).

HRMS (ESI⁺) m/z: calcd for C₄₄H₄₂N₁₀O₄Si: 401.1574 [M+2H]²⁺; found: 401.1574.

### [Production Example 6]

### <Synthesis of Bis(4-Aminomethylphenoxide) Silicon Phthalocyanine (pc6)>

A reaction product pc6 was synthesized as in the synthesis of pc4, except that 4-(aminomethyl)phenol was used instead of 4-aminobutanol.

### [Examples 1 to 3]

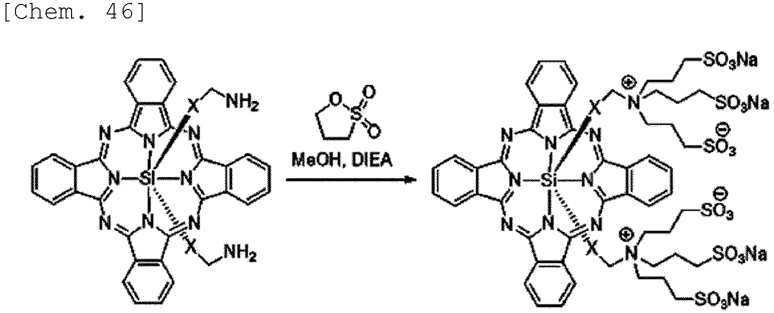

### [Example 1]

### <Synthesis of Bis{4-[Tris(3-Sulfopropyl)]Ammoniobutyloxide} Silicon Phthalocyanine (Compound 2)>

The pc4 (30 mg, 0.042 mmol), 1,3-propanesultone (123 mg, 1.0 mmol), and N,N-diisopropylethylamine (DIEA, 271 mg, 2.1 mmol) were dissolved in methanol (4 ml). The resulting mixture was stirred for 72 hours at 50°C in an argon atmosphere.

The product was cleaned and purified as in Reference Example 1 and then subjected to cation exchange as in Reference Example 1. Hereby, 12.9 mg (0.00084 mmol, yield as soda salt: 20%) of a compound 2 was prepared.

¹H NMR (400 MHz, CD₃OH): 6 -1.93 (t, J = 6.1 Hz, 4H), - 1.46 to -1.37 (m, 4H), -1.01 to -0.91 (m, 4H), 1.32 to 1.50 (m, 16H), 2.53 to 2.64 (m, 24H), 8.53 (dd, J = 2.9, 5.7 Hz, 8H), 9.79 (dd, J = 2.9, 5.6 Hz, 8H).

HRMS (ESI⁻): m/z calcd for C₅₈H₆₈N₁₀Na₃O_{2O}S₆Si: 1513.240 [M-Na]⁻; found: 1513.241.

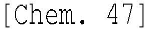

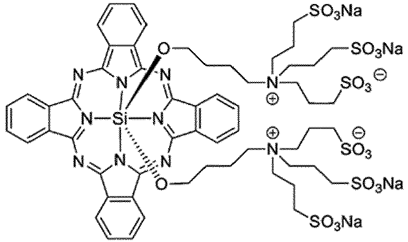

### [Example 2]

### <Synthesis of Bis({6-[Tris(3-Sulfopropyl)]Ammoniohexanoyl}Oxide) Silicon Phthalocyanine (Compound 3)>

The pc5 (75 mg, 0.094 mmol), 1,3-propanesultone (288 mg, 2.4 mmol), and DIEA (610 mg, 4.7 mmol) were dissolved in methanol (4 ml). The resulting mixture was stirred for 72 hours at 50°C in an argon atmosphere.

The product was cleaned and purified as in Reference Example 1 and then subjected to cation exchange as in Reference Example 1. Hereby, 31.0 mg (0.019 mmol, yield as soda salt: 20%) of a compound 3 was prepared.

¹H NMR (400 MHz, CD₃OD): δ -0.69 to -0.66 (m, 8H), -0.57 to -0.54 (m, 4H), 0.63 to 0.66 (m, 4H), 1.88 to 1.91 (m, 12H), 2.36 to 2.38 (m, 4H), 2.78 (t, J = 6.3 Hz, 12H), 3.13 to 3.18 (m, 12H), 8.57 (dd, J = 3.0, 6.0 Hz, 8H), 9.80 (dd, J = 3.0, 6.0 Hz, 8H) . HRMS (ESI⁻) : m/z calcd for C₆₂H₇₂N₁₀Na₃O₂₂S₆Si: 1597.2615 [M-Na]⁻; found: 1597.2615.

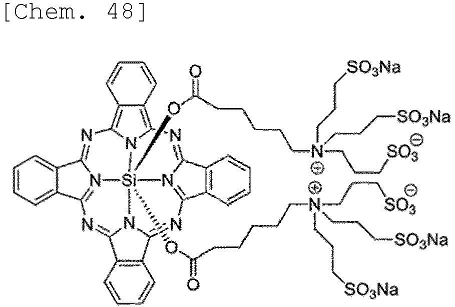

### [Example 3]

### <Synthesis of Bis{4-[Tris(3-Sulfopropyl)]Ammoniomethylphenoxide} Silicon Phthalocyanine (Compound 4)>

The pc6 (40 mg, 0.052 mmol), 1,3-propanesultone (153 mg, 1.3 mmol), and DIEA (336 mg, 2.6 mmol) were dissolved in methanol (4 ml). The resulting mixture was stirred for 72 hours at 50°C in an argon atmosphere.

The product was cleaned and purified as in Reference Example 1 and then subjected to cation exchange as in Reference Example 1. Hereby, 13.8 mg (0.0086 mmol, yield as soda salt: 7%) of a compound 4 was prepared.

¹H NMR (400 MHz, CD₃OD): δ 2.00 to 2.09 (m, 12H), 2.16 (s, 4H), 2.59 (d, J = 8.3 Hz, 4H), 2.70 to 2.81 (m, 24H), 5.84 (d, J = 8.3 Hz, 4H), 8.57 (dd, J = 2.8, 5.5 Hz, 8H), 9.81 (dd, J = 2.8, 5.5 Hz, 8H) . HRMS (ESI⁻): m/z calcd for C₆₄H₆₄N₁₀Na₃O₂₀S₆Si: 1581.2090 [M-Na]⁻; found: 1581.2101.

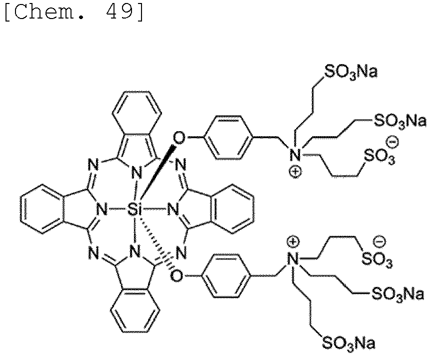

### [Example 4]

### <Synthesis of {3-[Tris(3-Sulfopropyl)]Ammoniopropyldimethylsilyl Oxide} Silicon Phthalocyanine (Compound 5)>

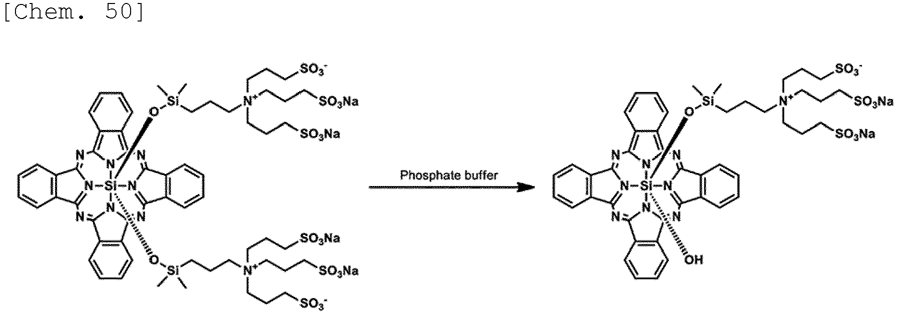

The compound 1 (27 mg, 16 µmol) prepared in Reference Example 1 was dissolved in an acidic phosphoric acid buffer solution (0.1 M, pH: 3.0, 1.8 ml). The resulting mixture was stirred for 1 hour at room temperature. Subsequently, a neutral phosphoric acid buffer solution (0.1 M, pH: 7.0, 1.8 ml) was added to the mixture. Then, rapid cooling was performed.

The product was cleaned and purified as in Reference Example 1 and then subjected to cation exchange as in Reference Example 1. Hereby, 5.4 mg (0.0046 mmol, yield as soda salt: 29%) of a compound 5 was prepared.

¹H NMR (400 MHz, CD₃OH): δ -2.80 (s, 6H), -2.12 (t, J = 8.3 Hz, 2H), -0.98 to -1.09 (m, 2H), 1.78 to 1.67 (m, 6H), 2.01 (t, J = 8.1 Hz, 2H), 2.82 to 2.72 (m, 12H), 8.48 (dd, J = 5.7, 3.0 Hz, 8H), 9.75 (dd, J = 5.7, 3.0 Hz, 8H).
HRMS (ESI-) m/z: [M-Na]- calcd for C₄₆H₄₇O₁₁N₉NaS₃Si₂, 1076.1999; found, 1076.2033

### [Examples 5 to 8]

### <Preparation of Calibration Curves>

For each of the compound 1 prepared in Reference Example 1 and the compounds 2 to 4 prepared in Examples 1 to 3, respectively, 30 µl of a 0.1-M phosphoric acid buffer solution (pH: 7.0) containing 0.2 to 10 µM of the compound was mixed with 30 µl of a 0.1-M phosphoric acid buffer solution (pH: 7.0) containing 10 µM of methylene blue, and 10 µl of the resulting mixture was injected into HPLC.

A calibration curve was prepared by calculating the areas of absorption peaks of the compounds 1 to 4 and methylene blue at specific concentrations and plotting the ratios between the peak areas of methylene blue and the compounds and the ratios between the concentrations of methylene blue and the compounds.

### <HPLC Conditions>

HPLC System: HPLC system (produced by Shimadzu Corporation) equipped with a reverse-phase column Inertsil ODS-3 (10 mm × 250 mm) (produced by GL Sciences).
Eluent A: 0.1-M triethylamine acetic acid buffer solution
Eluent B: Liquid mixture containing 99% acetonitrile and 1% water
A/B (Conditions for compounds 1, 3, and 4): A/B = 80/20 to 0/100 (10 min) A/B (Conditions for compound 2): A/B = 75/25 to 60/40 (5 min) to 0/100 (10 min)
Flow: 1 ml/min
Detection: 670 nm

### <Decomposition of Compound by X-Ray Irradiation>

For each of the compound 1 prepared in Reference Example 1 and the compounds 2 to 4 prepared in Examples 1 to 3, respectively, 1 ml of a 0.1-M phosphoric acid buffer solution (pH: 7.0) containing 0.5 µM of the compound was prepared. This buffer solution was used as an X-ray irradiation solution for the compound.

The X-ray irradiation solution for the compound was bubbled with Ar and then irradiated with X-rays at 6 MeV (20 Gy, 4.4 Gy/min) using LINAC (linear accelerator).

From the X-ray irradiation solution for the compound which had been irradiated with X-rays, 30 µl of a sample was taken and mixed with 30 µl of a 0.1-M phosphoric acid buffer solution (pH: 7.0) containing 10 µM of methylene blue, and 10 µl of the resulting mixture was injected into HPLC.

The peak area ratio was calculated on the basis of the areas of absorption peaks of the compound and methylene blue. The ratio between the concentrations of methylene blue and the compound was determined using the calibration curve. The concentration of the compound was calculated using the known concentration of the methylene blue. Thus, the residual ratio (Post/Pre(%)) of the compound was determined.

It was found that all of the compounds 1 to 4 were decomposed due to X-ray irradiation. Note that, the smaller the residual ratio (Post/Pre(%)), the higher the degree of progress of the decomposition (selectively decomposition of chemical bonds; dissociation of axial ligands) by X-rays.

Table 1 lists the results.

**[Table 1]**

| | Example 5 | Example 6 | Example 7 | Example 8 |
|---|---|---|---|---|
| | Compound 1 | Compound 2 | Compound 3 | Compound 4 |
| Chemical structure | 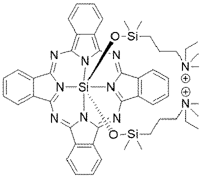 | 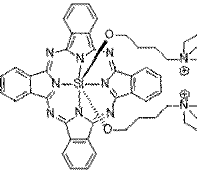 | 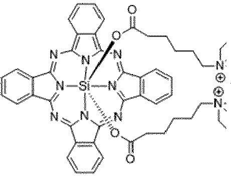 | 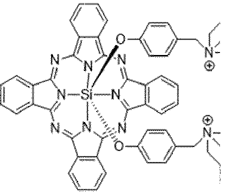 |
| Post/pre (%) | 52.5 | 46.8 | 62.1 | 74.8 |

### {Impact of pH}

### [Reference Examples 1 and 2]

In a phosphoric acid buffer solution having a pH of 6 or 7, 5 µM of the compound 2 was dissolved. While degassing was performed using Ar, the resulting mixture was irradiated with X-rays (6 MeV) using LINAC such that the amount of X-rays absorbed was 20 Gy. The sample irradiated with X-rays was analyzed using HPLC as in Example 5 to calculate the residual ratios of the compound 2 at the above pH values. The above calculation was conducted three times for each case, and the average was considered as the residual ratio (Post/Pre(%)) of the compound 2.

In Reference Example 1 where the compound 2 was dissolved in a phosphoric acid buffer solution having a pH of 6, the residual ratio (Post/Pre(%)) at which the compound 2 remained after the X-ray irradiation was 18.6%.

In Reference Example 2 where the compound 2 was dissolved in a phosphoric acid buffer solution having a pH of 7, the residual ratio (Post/Pre(%)) at which the compound 2 remained after the X-ray irradiation was 50.0%.

This confirms that, the lower the pH, the higher the likelihood of dissociation of the axial ligands.

### {Impact of Radical Species}

### [Reference Example 3]

Sodium formate was added to a 5-µM aqueous solution of the compound 2 as a radical scavenger. While degassing was performed using Ar, the resulting mixture was irradiated with X-rays (6 MeV) using LINAC such that the amount of X-rays absorbed was 2, 3, 4, or 5 Gy. Immediately after the irradiation, the degassing was stopped and the sample irradiated with X-rays was analyzed using HPLC as in Example 5 to calculate the residual ratios of the compound 2 under the above irradiation conditions. Note that, when the amount of X-rays emitted was 5 Gy, the amount of e⁻_{aq} (hydrated electrons) produced was 1.6 µM and the amount of CO₂⁻· (carbon dioxide anion radicals) produced was 1.6 µM. The irradiation and calculation of the residual ratio were conducted three times for each case, and the average was considered as the residual ratio (Post/Pre(%)) of the compound 2. Table 2 lists the results.

### [Reference Example 4]

The residual ratio (Post/Pre(%)) of the compound 2 was calculated as in Reference Example 3, except that the degassing was stopped after the Ar degassing had been maintained 30 minutes subsequent to the X-ray irradiation. Table 2 lists the results.

### [Reference Example 5]

The residual ratio (Post/Pre(%)) of the compound 2 was calculated as in Reference Example 3, except that, instead of sodium formate, sodium formate and nitrous oxide were used as a radical scavenger. Table 2 lists the results. Note that, when the amount of X-rays emitted was 5 Gy, the amount of e⁻_{aq} (hydrated electrons) produced was 0 µM and the amount of CO₂⁻· (carbon dioxide anion radicals) produced was 3.1 µM.

### [Reference Example 6]

The residual ratio (Post/Pre(%)) of the compound 2 was calculated as in Reference Example 5, except that the degassing was stopped after the Ar degassing had been maintained 30 minutes subsequent to the X-ray irradiation. Table 2 lists the results.

### [Reference Example 7]

The residual ratio (Post/Pre(%)) of the compound 2 was calculated as in Reference Example 3, except that the radical scavenger was not used. Table 2 lists the results.

**[Table 2]**

| Amount of X-rays absorbed | Reference example | | | | |
|---|---|---|---|---|---|
| | 3 | 4 | 5 | 6 | 7 |
| 2 Gy | 98.4% | 100% | 96.0% | 93.1% | 100% |
| 3 Gy | 93.3% | 49.9% | 90.4% | 85.4% | 99.4% |
| 4 Gy | 89.5% | 0.3% | 86.2% | 36.1% | 99.4% |
| 5 Gy | 8.8% | 0% | 8.3% | 4.8% | 100% |

The results obtained in Reference Example 3 to 6 confirm that, even in the case where the amount of X-ray (6 MeV) emitted is 10 Gy or less, that is, for example, 2 to 5 Gy, the selective decomposition of the chemical bonds of the compound 2 (dissociation of axial ligands) occurs due to the action of e⁻_{aq} (hydrated electrons) and/or CO₂⁻· (carbon dioxide anion radicals). It was also confirmed that continuing the Ar degassing subsequent to the irradiation enables the selective decomposition of the chemical bonds of the compound 2 to occur.

On the basis of the results obtained in Reference Examples 3 to 6, it is also considered that e⁻_{aq} (hydrated electrons) and/or CO₂⁻· (carbon dioxide anion radicals) contribute to the dissociation of the axial ligands of the compound 2. Since the residual ratio of the compound 2 decreased in the cases where the degassing was maintained, it is also considered that maintaining the degassing causes a radical chain reaction responsible for the dissociation of the axial ligands.

The knowledge obtained in Reference Examples 1 to 7 is quite useful for discovering a compound in which the selective decomposition of chemical bonds (dissociation of axial ligands) can be achieved with a smaller amount of irradiation.

## Claims

1. A method for decomposing a compound represented by Formula (1), the method comprising irradiating the compound with X-rays in a presence of an electron donor: 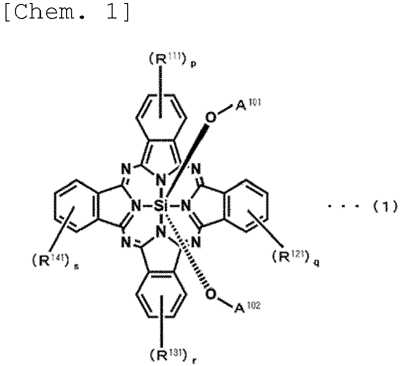 (in Formula (1), R¹¹¹, R¹²¹, R¹³¹, and R¹⁴¹ each independently represent a monovalent organic group; p, q, r, and s each independently represent an integer of 0 or 1 to 4; in the case where two or more R¹¹¹, R¹²¹, R¹³¹, and R¹⁴¹ are present, they may be identical to or different from one another or may be bonded to one another to form a ring; A¹⁰¹ represents a monovalent organic group; and A¹⁰² represents a hydrogen atom or a monovalent organic group).

2. The method for decomposing the compound according to Claim 1, wherein a product of the decomposition is one or more compounds selected from the group consisting of a compound represented by Formula (2), a compound represented by Formula (3), and a compound represented by Formula (4).
Formula (2): 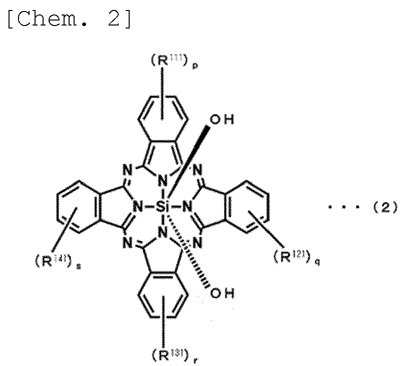 (in Formula (2), R¹, R², R³, R⁴, p, q, r, and s represent the same things as R¹, R², R³, R⁴, p, q, r, and s in Formula (1), respectively)
Formula (3):
A¹⁰¹-OH ... (3)
(in Formula (3), A¹⁰¹ represents the same thing as A¹⁰¹ in Formula (1))
Formula (4):
A¹⁰²-OH ... (4)
(in Formula (4), A¹⁰² represents the same thing as A¹⁰² in Formula (1))

3. The method for decomposing the compound according to Claim 1, wherein the X-rays have wavelengths of 1 pm to 2 nm and/or the X-rays have an irradiation energy of 1 keV to 100 MeV.

4. The method for decomposing the compound according to Claim 1 or 2,
wherein the A¹⁰¹ is a group represented by Formula (1a): 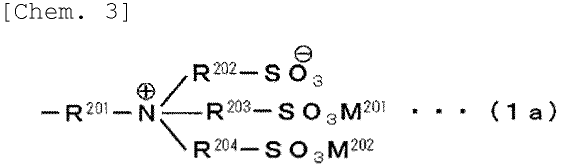 (in Formula (1a), R²⁰¹, R²⁰², R²⁰³, and R²⁰⁴ each independently represent a divalent organic group and may be identical to or different from one another; and M²⁰¹ and M²⁰² each independently represent a monovalent cation and may be identical to or different from one another), and
wherein the A¹⁰² is a hydrogen atom or a group represented by Formula (1b): 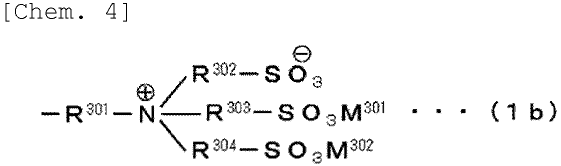 (in Formula (1b), R³⁰¹, R³⁰², R³⁰³, and R³⁰⁴ each independently represent a divalent organic group and may be identical to or different from one another; and M³⁰¹ and M³⁰² each independently represent a monovalent cation and may be identical to or different from one another).

5. The method for decomposing the compound according to any one of Claims 1 to 3, wherein at least one of the R¹¹¹, R¹²¹, R¹³¹, and R¹⁴¹ is a group represented by Formula (1c) :
-L-Q (1c)
(in Formula (1c), L represents a divalent organic group, and Q represents a reactive group responsible for addition to a probe).

6. The method for decomposing the compound according to Claim 1,
wherein the A¹⁰¹ is any one of groups represented by Formulae (1a₁), (1a₂), and (1a₃),
Formula (1a₁): 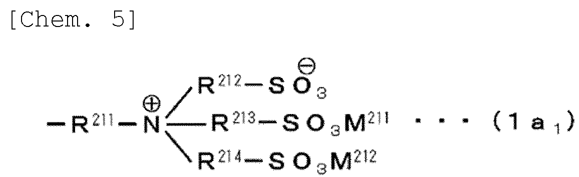 (in Formula (1a₁), R²¹¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R²¹², R²¹³, and R²¹⁴ each independently represent a divalent aliphatic hydrocarbon group having 1 to 20 carbon atoms and may be identical to or different from one another; and M²¹¹ and M²¹² each independently represent an alkali metal ion and may be identical to or different from one another)
Formula (1a₂): 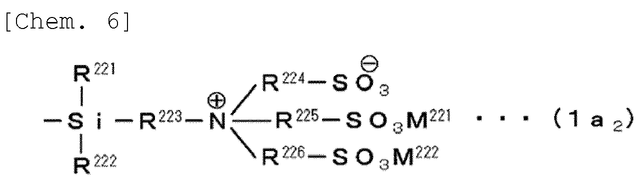 (in Formula (1a₂), R²²¹ and R²²² each independently represent a monovalent aliphatic hydrocarbon group having 1 to 20 carbon atoms or a monovalent aromatic hydrocarbon group having 6 to 30 carbon atoms; R²²³ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R²²⁴, R²²⁵, and R²²⁶ each independently represent a divalent aliphatic hydrocarbon group having 1 to 20 carbon atoms and may be identical to or different from one another; and M²²¹ and M²²² each independently represent an alkali metal ion and may be identical to or different from one another)
Formula (1a₃): 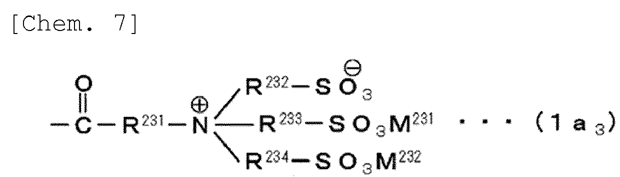 (in Formula (1a₃), R²³¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R²³², R²³³, and R²³⁴ each independently represent a divalent aliphatic hydrocarbon group having 1 to 20 carbon atoms and may be identical to or different from one another; and M²³¹ and M²³² each independently represent an alkali metal ion and may be identical to or different from one another), and
wherein the A¹⁰² is any one of a hydrogen atom and groups represented by Formulae (1b₁), (1b₂), and (1b₃),
Formula (1b₁): 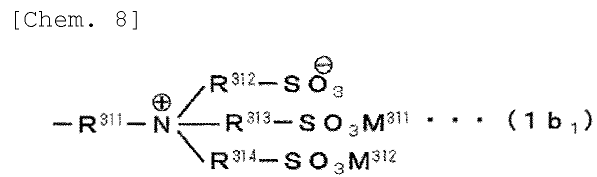 (in Formula (1b₁), R³¹¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R³¹², R³¹³, and R³¹⁴ each independently represent a divalent aliphatic hydrocarbon group having 1 to 20 carbon atoms and may be identical to or different from one another; and M³¹¹ and M³¹² each independently represent an alkali metal ion and may be identical to or different from one another)
Formula (1b₂): 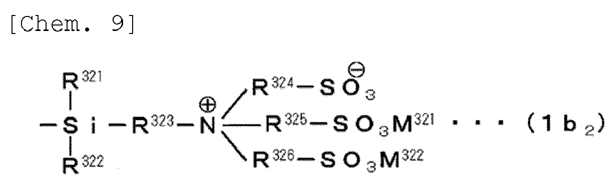 (in Formula (1b₂), R³²¹ and R³²² each independently represent a monovalent aliphatic hydrocarbon group having 1 to 20 carbon atoms or a monovalent aromatic hydrocarbon group having 6 to 30 carbon atoms; R³²³ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R³²⁴, R³²⁵, and R³²⁶ each independently represent a divalent aliphatic hydrocarbon group having 1 to 20 carbon atoms and may be identical to or different from one another; and M³²¹ and M³²² each independently represent an alkali metal ion and may be identical to or different from one another)
Formula (1b₃): 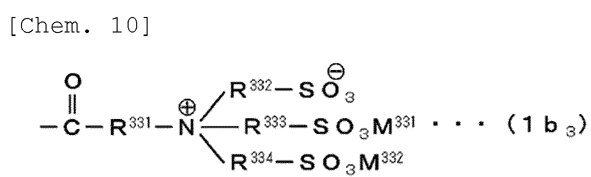 (in Formula (1b₃), R³³¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R³³², R³³³, and R³³⁴ each independently represent a divalent aliphatic hydrocarbon group having 1 to 20 carbon atoms and may be identical to or different from one another; and M³³¹ and M³³² each independently represent an alkali metal ion and may be identical to or different from one another).

7. The method for decomposing the compound according to any one of Claims 1 to 6, the method being conducted in a presence of hydrated electrons and/or carbon dioxide anion radicals.

8. The method for decomposing the compound according to any one of Claims 1 to 7, wherein an amount of X-rays absorbed is less than 10 Gy.

9. A compound represented by Formula (3): 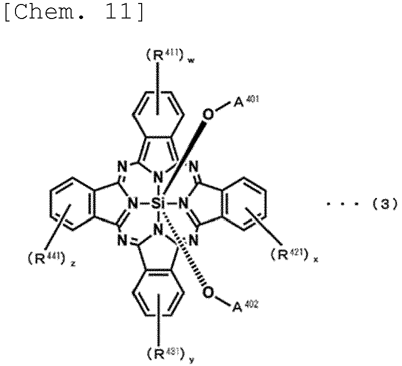 (in Formula (3),
R⁴¹¹, R⁴²¹, R⁴³¹, and R⁴⁴¹ each independently represent a monovalent organic group; w, x, y, and z each independently represent an integer of 0 or 1 to 4; in the case where two or more R⁴¹¹, R⁴²¹, R⁴³¹, and R⁴⁴¹ are present, they may be identical to or different from one another or may be bonded to one another to form a ring;
A⁴⁰¹ represents any one of groups represented by Formulae (3a₁), (3a₂), and (3a₃),
Formula (3a₁): 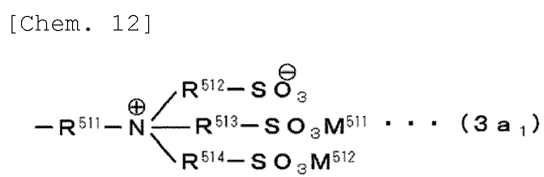 (in Formula (3a₁), R⁵¹¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁵¹², R⁵¹³, and R⁵¹⁴ each independently represent a divalent aliphatic hydrocarbon group having 1 to 20 carbon atoms and may be identical to or different from one another; and M⁵¹¹ and M⁵¹² each independently represent an alkali metal ion and may be identical to or different from one another)
Formula (3a₂): 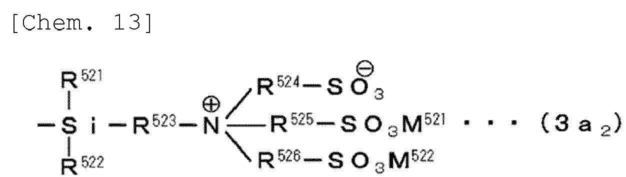 (in Formula (3a₂), R⁵²¹ and R⁵²² each independently represent a monovalent aliphatic hydrocarbon group having 1 to 20 carbon atoms or a monovalent aromatic hydrocarbon group having 6 to 30 carbon atoms; R⁵²³ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁵²⁴, R⁵²⁵, and R⁵²⁶ each independently represent a divalent aliphatic hydrocarbon group having 1 to 20 carbon atoms and may be identical to or different from one another; and M⁵²¹ and M⁵²² each independently represent an alkali metal ion and may be identical to or different from one another)
Formula (3a₃): 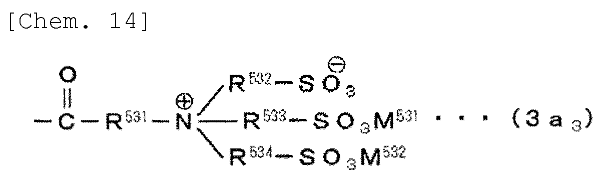 (in Formula (3a₃), R⁵³¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁵³², R⁵³³, and R⁵³⁴ each independently represent a divalent aliphatic hydrocarbon group having 1 to 20 carbon atoms and may be identical to or different from one another; and M⁵³¹ and M⁵³² each independently represent an alkali metal ion and may be identical to or different from one another); and
A⁴⁰² represents any one of a hydrogen atom and groups represented by Formulae (3b₁), (3b₃), and (3b₃),
Formula (3b₁): 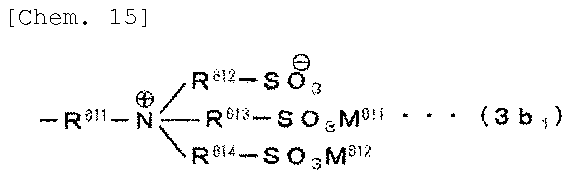 (in Formula (3b₁), R⁶¹¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁶¹², R⁶¹³, and R⁶¹⁴ each independently represent a divalent aliphatic hydrocarbon group having 1 to 20 carbon atoms and may be identical to or different from one another; and M⁶¹¹ and M⁶¹² each independently represent an alkali metal ion and may be identical to or different from one another)
Formula (3b₂): 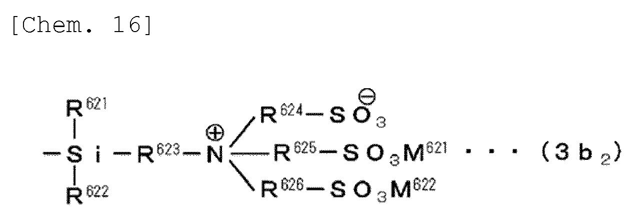 (in Formula (3b₂), R⁶²¹ and R⁶²² each independently represent a monovalent aliphatic hydrocarbon group having 1 to 20 carbon atoms or a divalent aromatic hydrocarbon group having 6 to 30 carbon atoms; R⁶²³ represents -CH₂-, -CH₂-CH₂-, -CH(CH₃) CH₂-, -CH₂CH(CH₃)-, a divalent aliphatic hydrocarbon group having 4 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁶²⁴, R⁶²⁵, and R⁶²⁶ each independently represent a divalent aliphatic hydrocarbon group having 1 to 20 carbon atoms and may be identical to or different from one another; and M⁶²¹ and M⁶²² each independently represent an alkali metal ion and may be identical to or different from one another)
Formula (3b₃): 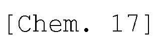 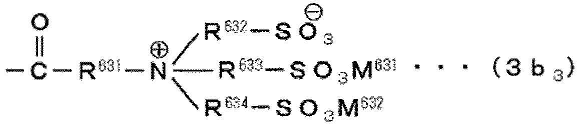 (in Formula (3b₃), R⁶³¹ represents a divalent aliphatic hydrocarbon group having 1 to 20 carbon atoms, a divalent alicyclic hydrocarbon group having 3 to 20 carbon atoms, a divalent aromatic hydrocarbon group having 6 to 30 carbon atoms, a divalent aromatic-aliphatic hydrocarbon group having 7 to 30 carbon atoms, or a divalent alicyclic-aliphatic hydrocarbon group having 4 to 20 carbon atoms; R⁶³², R⁶³³, and R⁶³⁴ each independently represent a divalent aliphatic hydrocarbon group having 1 to 20 carbon atoms and may be identical to or different from one another; and M⁶³¹ and M⁶³² each independently represent an alkali metal ion and may be identical to or different from one another)).
